(19) Europäisches Patentamt
European Patent Office
Office européen des brevets

(11) EP 3 202 314 A1

(12) EUROPEAN PATENT APPLICATION

(43) Date of publication:
**09.08.2017 Bulletin 2017/32**

(21) Application number: **17153263.3**

(22) Date of filing: **26.01.2017**

(51) Int Cl.:
***A61B 5/02*** *(2006.01)*
***A61B 5/0205*** *(2006.01)*
***A61B 5/024*** *(2006.01)*
***A61B 5/11*** *(2006.01)*
***A61B 5/00*** *(2006.01)*
*A61B 5/05* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.02.2016 JP 2016018224**

(71) Applicant: **FUJITSU LIMITED**
**Kawasaki-shi Kanagawa 211-8588 (JP)**

(72) Inventor: **Yamaji, Takayuki**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**

# (54) SENSOR INFORMATION PROCESSING APPARATUS

(57) A detected signal of a heartbeat sensor (21) and a detected signal of an inertial sensor (22) are received by a processor (31 or 23). The processor estimates a heart rate in a case where an exercise intensity obtain from the detected signal of the inertial sensor (22) is equal to or more than a threshold value, based on a relation between the exercise intensity and a heart rate obtained from the detected signal of the heartbeat sensor (21) during a period in which the exercise intensity obtained from the detected signal of the inertial sensor (22) is less than the threshold value.

FIG. 1

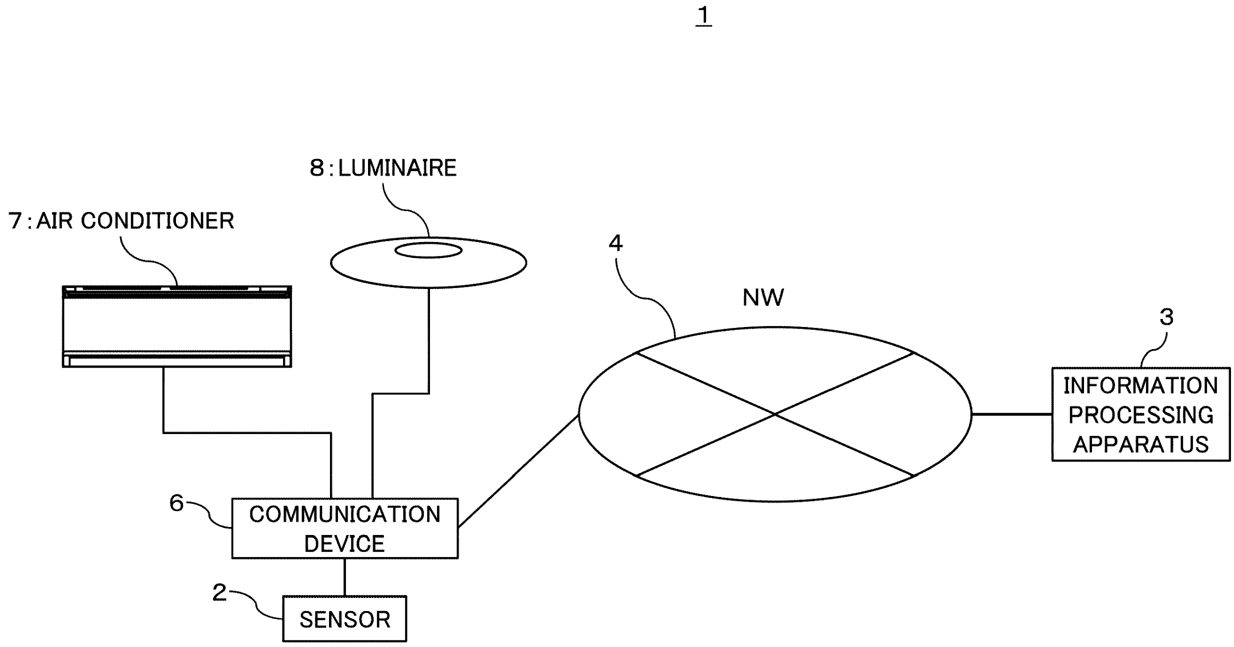




Printed by Jouve, 75001 PARIS (FR)

## Description

FIELD

The embodiment (s) discussed herein is related to a sensor information processing apparatus.

BACKGROUND

There is a technique of detecting a heartbeat of a biological object (e.g. a person) by using a heartbeat sensor. A bandpass filter (BPF) is applied to a detected signal of the heartbeat sensor to detect a signal component corresponding to the heartbeat from the detected signal of the heartbeat sensor.

(RELATED ART DOCUMENTS LIST)

Patent Document 1JP2003-334269 A

Patent Document 2JP11-192217 A

Patent Document 3JP2014-94043 A

Patent Document 4JP2014-039666 A

During a rest time of a biological object, a noise component corresponding to a body motion of the biological object is hardly mixed in a detected signal of a heartbeat sensor. However, when the biological object makes a body motion during an activity or an exercise of the biological object, a body motion derived noise component is likely to be mixed in a detected signal of the heartbeat sensor. Hence, when BPF characteristics are not appropriately set, a detection accuracy of a heartbeat derived signal component (which maybe referred to as a "heartbeat signal") lowers.

A heart rate of a person tends to be high generally when an exercise intensity is high. Therefore, according to the general tendency, it may also be possible to estimate an approximate heart rate during an activity or an exercise of the person and determine BPF characteristics.

However, a change in a heart rate (e.g. how the heart rate increases or decreases) associated with a body motion during an activity or an exercise of a person is an individual difference. Even when BPF characteristics are set based on a generalized tendency without taking into account the individual difference in the change in the heart rate, there is a concern that the BPF does not have characteristics which are appropriate to detect a heartbeat derived signal component (which may be referred to as a "heartbeat signal"). Hence, a detection accuracy of a heartbeat signal lowers.

SUMMARY

According to one aspect, one of objects of the technique discussed herein is to improve a detection accuracy of a heartbeat signal obtained by a heartbeat sensor for each individual.

In one aspect, a sensor information processing apparatus may include a receiver and a processor. The receiver may receive a detected signal of a heartbeat sensor and a detected signal of an inertial sensor. The processor may estimate a heart rate in a case where an exercise intensity obtain from the detected signal of the inertial sensor is equal to or more than a threshold value, based on a relation between the exercise intensity and a heart rate obtained from the detected signal of the heartbeat sensor during a period in which the exercise intensity obtained from the detected signal of the inertial sensor is less than the threshold value.

Further, in another aspect, a sensor information processing apparatus may include a receiver and a processor. The receiver may receive a detected signal of a heartbeat sensor and a detected signal of an inertial sensor. The processor may estimate a heart rate in a case where the detected signal of the inertial sensor is equal to or more than a threshold value, based on a relation between the detected signal of the inertial sensor and the detected signal of the heartbeat sensor obtained during a period in which the detected signal of the inertial sensor is less than the threshold value.

According to one aspect, it is possible to improve a detection accuracy of a heartbeat signal obtained by the heartbeat sensor for each individual.

BRIEF DESCRIPTION OF DRAWINGS

FIG. 1 is a block diagram illustrating an example of a sensor system according to an embodiment;
FIGS. 2 and 3 are block diagrams illustrating configuration examples of a vital sensor according to an embodiment;
FIG. 4 is a block diagram illustrating a configuration example of an information processing apparatus according to an embodiment;
FIG. 5 is a diagram illustrating an example of a result of a heart rate measurement according to an embodiment;
FIG. 6 is a diagram illustrating an example of a temporal change in a heart rate (estimated value and actual measured value) and an exercise intensity (METs value) according to an embodiment;
FIG. 7 is a flowchart illustrating an operation example of a sensor system according to a first example;
FIG. 8 is a flowchart illustrating an example of an exercise intensity calculation process illustrated in FIG. 7;
FIG. 9 is a diagram illustrating an example of a relation between a walking cadence and a walking speed according to an embodiment;
FIG. 10 is a diagram illustrating an example of a relation between a walking speed and an exercise intensity (METs value) according to an embodiment;
FIG. 11 is a flowchart illustrating an example of the heart rate estimation process illustrated in FIG. 7;
FIG. 12 is a flowchart illustrating a generation example of exercise intensity-to-heart rate information according to one embodiment;
FIGS. 13 and 14 are diagrams illustrating examples of a relation between an exercise intensity (METs value) and a heart rate according to one embodiment;
FIG. 15 is a diagram illustrating an example of a relation between an exercise intensity (METs value), and a walking speed, an age estimated heart rate and a measured heart rate according to one embodiment;
FIG. 16A is a diagram illustrating an example of a relation between an exercise intensity (METs value), and a walking speed, an age estimated heart rate, a measured heart rate and a maximum heart rate (constant S) according to one embodiment;
FIG. 16B is a diagram illustrating an example of a relation between an exercise intensity (METs value), and a walking speed, an age estimated heart rate, a measured heart rate, a maximum heart rate (constant S) and an estimated heart rate according to one embodiment;
FIG. 17 is a diagram illustrating an example of a relation between an exercise intensity (METs value) and a constant (S) according to one embodiment;
FIG. 18 is a diagram illustrating an example of a relation between an exercise intensity (illustratively, a walking speed) and a measured heart rate in a low load area according to one embodiment;
FIG. 19 is a diagram illustrating an example of a relation between an exercise intensity (illustratively, a walking speed) and an estimated heart rate in a high load area according to one embodiment;
FIG. 20 is a diagram illustrating an example of a relation obtained by synthesizing the relations in the low load area and the high load area illustrated in FIGS. 17 and 18;
FIG. 21 is a flowchart illustrating an operation example of a sensor system according to a second embodiment;
FIG. 22 is a diagram illustrating a setting example of a BPF according to one embodiment;
FIG. 23 is a diagram illustrating an example of a passband of the BPF according to one embodiment;
FIG. 24 is a diagram illustrating a setting example of a bandwidth of a BPF according to one embodiment;
FIGS. 25 and 26 are diagrams illustrating examples of distributions of the heart rate according to an embodiment;
FIGS. 27 and 28 are diagrams illustrating examples of a statistical process of the heart rate according to an embodiment;
FIGS. 29 and 30 are diagrams illustrating setting examples of a bandwidth of a BPF according to one embodiment; and
FIG. 31 is a diagram illustrating an example of bandwidth information of the BPF according to an embodiment.

DESCRIPTION OF EMBODIMENTS

Hereinafter, an exemplary embodiment (s) will be described with reference to the drawings. However, the embodiment (s) described below is merely an example and not intended to exclude an application of various modifications or techniques which are not explicitly described below. Further, various exemplary aspects described below may be appropriately combined and carried out. Elements or components assigned the same reference numeral in the drawings used for the following embodiment(s) will represent identical or similar elements or components unless otherwise specified.

FIG. 1 is a block diagram illustrating an example of a sensor system according to an embodiment. A sensor system 1 illustrated in FIG. 1 may illustratively include a vital sensor 2, an information processing apparatus 3 and a network (NW) 4.

The vital sensor 2 may illustratively be connected to the network 4 through a communication device 6, and may be available to communicate with the information processing apparatus 3 through the network 4.

The vital sensor 2 is available to sense information of a biological object (hereinafter, may be referred to as "vital information"). The biological object is an example of a sensing target. The term of "sensing" may be referred to as "detection" or "measurement".

A non-restrictive example of the "vital information" is information indicative of a heartbeat of a biological object. Since a blood vessel of the biological object pulsates according to a heartbeat, the information indicative of the heartbeat may be considered as being equivalent to information indicative of a pulsebeat.

The heartbeat or the pulsebeat of the biological object can be illustratively acquired as a change in an electromagnetic wave, a pressure or sound corresponding to a heartbeat.

For example, when a blood vessel of a finger or an earlobe is irradiated with light such as infrared rays, the reflected light would be change periodically according to a rhythmical change in a bloodstream and light absorption characteristics. Hence, a heartbeat or a pulsebeat can be optically measured as a fluctuation of reflected light according to a change in a bloodstream.

Alternatively, when a biological object is irradiated with a radio wave such as a microwave, a rhythmical motion would occur in a surface of a biological object (e.g. skin) according to a heartbeat. Therefore, a distance between the skin and a radio wave transmission source changes according to the motion, and changes due to a Doppler effect would occur in a reflected wave. Hence, it is also possible to measure a heartbeat or a pulsebeat as a fluctuation caused by the Doppler effect of the reflected wave irradiated on the biological object.

Further, when a heart is contracted and relaxed rhythmically, a pressure of a blood vessel (hereinafter, may be referred to as a "blood pressure") would also be fluctuated. Therefore, it is also possible to measure a heartbeat or a pulsebeat as a rhythmical fluctuation of a blood pressure by using a pressure sensor or a piezoelectric sensor.

Furthermore, it is also possible to measure a heartbeat as a change in an electric potential or a change in sound of a heart muscle corresponding to a heart pulse by using an electrocardiograph or a phonocardiograph.

A sensor available to measure a heartbeat using any one of the above measurement schemes may be referred to as a "heartbeat sensor" or a "heartbeat meter". As described above, since the information indicative of a "heartbeat" may be considered as being equivalent to the information indicative of a "pulsebeat" in some cases, the "heartbeat sensor" or the "heartbeat meter" may be referred to as a "pulsebeat sensor" or a "pulsebeat meter".

In the present embodiment, an example where a "wireless sensor" available to measure a heartbeat by using the Doppler effect is applied to the vital sensor 2 as an example of the heartbeat sensor will be described for descriptive purposes. The "wireless sensor" may be referred to as a "microwave sensor", an "RF (Radio Frequency) sensor" or a "Doppler sensor".

The vital sensor 2 may be illustratively attached in contact with a skin of a human body or may be attached to clothes of the human body. The vital sensor 2 does not need to be attached to a human body by way of strictly fixing (may be referred to as "restraining") . It may be allowed to occur a relative motion between the vital sensor 2 and a human body according to a mismatch between motions of the clothes and a human body surface.

For example, the vital sensor 2 may be attached to a human body such that the vital sensor 2 is allowed to be moved in one of three-dimensional directions relative to a human body. Illustratively, the vital sensor 2 may be put in a pocket of clothes such as a breast pocket of a jacket or may be attached on the clothes by using an attachment tool such as a harness.

Next, the communication device 6 illustrated in FIG. 1 is available to transmit a sensing result (e.g. the information indicative of the heartbeat) of the vital sensor 2 to the information processing apparatus 3 through the network 4, for example. Hence, the communication device 6 may be connected with the network 4 using a wired cable or radio.

In other words, the communication device 6 may be provided with a communication interface (IF) which supports one or both of wireless and wired communications. Illustratively, a communication scheme based on the LTE (Long Term Evolution) or the LTE-Advanced of 3GPP (3rd Generation Partnership Project) is applicable to the wireless communication of the communication device 6.

Further, satellite communications may be applied to the wireless communication of the communication device 6. When the satellite communication is applied, the communication device 6 may be able to communicate with the information processing apparatus 3 through a communication satellite without being routed through the network 4.

The sensing result of the vital sensor 2 may include not only vital information but also information indicative of a result of an arithmetic operation or determination, which is obtained based on the vital information. The sensing result maybe referred to as "sensor information" or "sensor data" for descriptive purposes.

The communication device 6 may be externally attached to the vital sensor 2 as illustrated in FIG. 1, or may be built in the vital sensor 2. The communication device 6 externally attached to the vital sensor 2 may be, for example, a device carried by a person attached with the vital sensor 2. The person attached with the vital sensor 2 may be referred to as a "user", a "subject" or an "observed person" for descriptive purposes.

The communication device 6 carried by the user may be illustratively a mobile telephone (which may include a smartphone), a notebook PC or a tablet PC. The "PC" is an abbreviation of a "personal computer".

A wired connection or a wireless connection may be applied to a connection between the vital sensor 2 and the communication device 6. In other words, the vital sensor 2 may be provided with a communication IF which supports one or both of wireless and wired communications. The "WiFi (Wireless Fidelity)" (registered trademark) or "Bluetooth" (registered trademark) may also be applied to the wireless connection.

The communication device 6 externally attached to the vital sensor 2 may be a router or a network switch. As illustrated in FIG. 1, the communication device 6 may be communicably connected with an air conditioner 7 and an luminaire 8 so that the air conditioner 7 and the luminaire 8 are able to communicate with the information processing apparatus 3 through the network 4.

The network 4 may be illustratively a WAN (Wide Area Network), a LAN (Local Area Network) or the Internet. Further, the network 4 may include a wireless access network. The wireless access network may be compliant with the above-described LTE or LTE-Advanced.

The information processing apparatus 3 receives the sensor information of the vital sensor 2 through the network 4 (or may be through the communication satellite), and processes the received sensor information. Hence, the information processing apparatus 3 may be referred to as the sensor information processing apparatus 3.

Processing the sensor information may include storing and managing the sensor information, and estimating a heart rate of the user based on the sensor information. Hence, the information processing apparatus 3 is available to monitor an activity status of the user, for example. In other words, the sensor system 1 can provide a user "monitoring (or watching) function".

Managing the sensor information may include compiling the sensor information in a database (DB). The data compiled in the DB may be referred to as "cloud data" or "big data".

The information processing apparatus 3 may be illustratively realized by one or a plurality of servers. In other words, the sensor information obtained by the vital sensor 2 may be processed or managed by a single server or may be distributedly processed or managed by a plurality of servers in the information processing apparatus 3. The server may correspond to a cloud server provided in a cloud data sensor, for example.

The information processing apparatus 3 may be communicably connected with the vital sensor 2 without being routed through the network 4. For example, the information processing apparatus 3 may be available to directly receive the sensor information from the vital sensor 2 through a wired cable or by radio.

(Configuration example of vital sensor 2)

Next, the configuration example of the vital sensor 2 will be described with reference to FIGS. 2 and 3. As illustrated in FIGS. 2 and 3, the vital sensor 2 may illustratively include a wireless sensor 21 which is an example of a heartbeat sensor, an inertial sensor 22, a processor 23, a memory 24 and a communication IF 25.

The vital sensor 2 may be referred to as the sensor unit 2. Hereinafter, the vital sensor 2 or the sensor unit 2 will be simply referred to as the "sensor 2" for descriptive purposes.

As illustrated in FIG. 3, the wireless sensor 21, the inertial sensor 22, the processor 23, the memory 24 and the communication IF 25 may be illustratively connected to a bus 26 to communicate with each other through the processor 23.

The wireless sensor 21 may be a Doppler sensor, and illustratively performs a phase detection on a radio wave transmitted to a space and a reflected wave of the transmitted radio wave to generate a beat signal. The beat signal may be supplied as an output signal of the wireless sensor 21 to the processor 23.

As illustrated in FIG. 2, the wireless sensor 21 may include, for example, an antenna 211, a local oscillator (OSC) 212, a MCU (Micro Control Unit) 213, a detection circuit 214, an operational amplifier (OP) 215 and a power supply unit (or a power supply circuit) 216.

The antenna 211 transmits to the space a radio wave having an oscillation frequency generated by the OSC 212, and receives a reflected wave of the transmitted radio wave reflected by the user positioned in the space. In an example of FIG. 2, the antenna 211 is shared by a transmission and a reception but a transmission antenna and a reception antenna may be provided individually.

The OSC 212 illustratively oscillates in response to a control of the MCU 213 to output a signal with a predetermined frequency (which may be referred to as a "local signal" for descriptive purposes). The local signal is transmitted as a transmission radio wave from the antenna 211, and is inputted to the detection circuit 214.

The oscillation frequency of the OSC 212 (in other words, a frequency of a radio wave transmitted by the wireless sensor 21) may be illustratively a frequency in a microwave band. The microwave band may be illustratively a 2.4 GHz band or a 24 GHz band.

These frequency bands are examples whose indoor use is authorized by the Radio Act in Japan. Frequency bands which are not regulated by the Radio Act may be used for a transmission radio wave of the wireless sensor 21.

The MCU 213 illustratively controls an oscillating operation of the OSC 212 in response to a control of the processor 23.

The detection circuit 214 illustratively performs the phase detection on the reflected wave received by the antenna 211 and the local signal (in other words, the transmission radio wave) from the OSC 212 to output the beat signal. The detection circuit 214 may be replaced with a mixer which mixes a transmission radio wave and a reflected wave. The mixing performed by the mixer may be considered as being equivalent to the phase detection.

In this regard, a change in an amplitude and a change in a frequency occur in the beat signal obtained by the detection circuit 214 due to the Doppler effect according to a heartbeat of the user. In other words, the beat signal includes information indicative of the heartbeat of the user.

The operational amplifier 215 illustratively amplifies the beat signal outputted from the detection circuit 214. The amplified beat signal is inputted to the processor 23.

The power supply unit 216 illustratively supplies drive power to the MCU 213, the detection circuit 214 and the operational amplifier 215.

Meanwhile, the inertial sensor 22 may illustratively detect a motion of the sensor unit 2. The inertial sensor 22 may be an acceleration sensor or a gyroscope. Any one of piezoelectric type and capacitive type sensors may be illustratively applied to the acceleration sensor. Any one of a spin rotor (flywheel) type, an optical type and a vibrating structure type sensors may be applied to the gyroscope.

The inertial sensor 22 may include one or more of detection axes. A gravity component in a direction along one of the detection axes may be detected as an "acceleration" component, for example. A detected signal of the inertial sensor 22 may be inputted to the processor 23.

The processor 23 is an example of an arithmetic processing apparatus with a capability of arithmetic processing. The arithmetic processing apparatus may be referred to as an arithmetic processing device or an arithmetic processing circuit. An integrated circuit (IC) such as an MPU (Micro Processing Unit) or a DSP (Digital Signal Processor) may be illustratively applied to the processor 23 which is an example of the arithmetic processing apparatus. The "processor" may be referred to as a "processing unit", a "controller" or a "computer".

The processor 23 is available to detect the heartbeat of the user based on the detected signal of the wireless sensor 21. A filter may be illustratively applied to the detected signal of the wireless sensor 21 to detect a heartbeat derived signal component (which may be referred to as a "heartbeat component" or a "heartbeat signal") from the detected signal of the wireless sensor 21. The filter of a non-restrictive example may be a bandpass filter (BPF). The processor 23 may determine a state related to a sleep of the user based on the detected heartbeat.

The detected signal of the inertial sensor 22 may be used to control filter characteristics (illustratively, a pass center frequency and passband width) of the above BPF. Controlling the filter characteristics may be considered as controlling a target frequency band to be processed in the detected signal of the wireless sensor 21. An example of a control of the filter characteristics will be described below.

The "pass center frequency" and the "passband width" of the BPF may be referred to simply as a "center frequency" and a "bandwidth", respectively.

The detected signal of the wireless sensor 21 and the detected signal of the inertial sensor 22 may be both referred to as a "detected value" or an "output value". For descriptive purposes, the detected value of the wireless sensor 21 may be referred to as a "wireless sensor value" and the detected value of the inertial sensor 22 may be referred to as an "inertial sensor value".

Further, the above-described detection of the heartbeat and control of the filter characteristics may be performed by a processor 31 of the information processing apparatus 3 (described below with reference to FIG. 4) instead of the processor 23 of the sensor unit 2.

Next, in FIG. 3, the memory 24 is an example of a storage unit or a storage medium provided in the sensor unit 2, and may be a RAM (Random Access Memory) or a flash memory.

A program and data read and used by the processor 23 to operate may be stored in the memory 24. The "program" may be referred to as a "software" or an "application" . The "data" may include data generated according to operations of the processor 23.

The communication IF 25 is an example of a communication unit of the sensor unit 2, and is illustratively connected with the communication device 6 (see FIG. 1) and enables communication with the information processing apparatus 3 through the network 4.

For example, the communication IF 25 may transmit the detected signals of the wireless sensor 21 and the inertial sensor 22, and transmit information obtained based on one or both of the detected signals to the information processing apparatus 3.

In other words, the sensor information transmitted from the vital sensor 2 to the information processing apparatus 3 may include measured values of the wireless sensor 21 and the inertial sensor 22, or may include information obtained based on one or both of the measured values.

The communication IF 25 may be connected with the information processing apparatus 3 to directly communicate with the information processing apparatus 3 without being routed through the communication device 6 and/or the network 4.

(Configuration example of information processing apparatus 3)

Next, the configuration example of the information processing apparatus 3 illustrated in FIG. 1 will be described with reference to FIG. 4. As illustrated in FIG. 4, the information processing apparatus 3 may illustratively include the processor 31, a memory 32, a storage device 33, a communication interface (IF) 34 and a peripheral IF 35.
The processor 31, the memory 32, the storage device 33, the communication IF 34 and the peripheral IF 35 may be illustratively connected to a communication bus 36 to communicate with each other through the processor 31.
The processor 31 is an example of an arithmetic processing apparatus which has a capability of arithmetic processing. The arithmetic processing apparatus may be referred to as an arithmetic processing device or an arithmetic processing circuit. An IC such as a CPU or a MPU or a DSP may be illustratively applied to the processor 31 which is an example of the arithmetic processing apparatus.
The processor 31 illustratively controls an entire operation of the information processing apparatus 3. The control performed by the processor 31 may include a control for communication performed through the network 4. By controlling the communication, the air conditioner 7 and the luminaire 8 may be remotely controlled through the network 4, for example.
Illustratively, the processor 31 may detect the heartbeat or control the filter characteristics as described above based on the sensor information of the vital sensor 2, which is received by the communication IF 34.
Further, the processor 31 may illustratively generate a control signal to control a spatial environment in which the user of the vital sensor 2 is positioned, such as a control signal to control operations of the air conditioner 7 and the luminaire 8.
The control signal may be illustratively generated based on a heart rate of the user detected based on the sensor information obtained from the vital sensor 2 and a state related to a sleep of the user estimated or determined based on the heart rate.
The control signal generated by the processor 31 may be illustratively transmitted to the air conditioner 7 and the luminaire 8 through the communication IF 34.
The memory 32 is an example of a storage medium, and may be a RAM or a flash memory. A program and data read and used by the processor 31 to operate may be stored in the memory 32.
The storage device 33 may store various pieces of data and programs. A hard disk drive (HDD), a solid state drive (SSD) or a flash memory may be applied to the storage device 33.
The data stored in the storage device 33 may illustratively include the sensor information of the sensor 2 received by the communication IF 34, the heart rate detected based on the sensor information, and the state related to the sleep of the user estimated or determined based on the heart rate.
The data stored in the storage device 33 may be optionally compiled in a database (DB). The data compiled in the DB may be referred to as "cloud data" or "big data". The storage device 33 and the memory 32 may be collectively referred to as a "storage unit 30" of the information processing apparatus 3.
The programs stored in the storage device 33 may include a program to execute processes described with reference to FIGS. 7 and 21.
The program to execute the processes described below with reference to FIGS. 8 and 14 may be referred to as a "sensor information processing program" for descriptive purposes.
All or part of program codes configuring a program may be stored in the storage unit or may be described as a part of an operating system (OS).
The program and the data may be recorded in a computer-readable non-transitory recording medium to be provided. Examples of the recording medium include flexible disks, CD-ROMs, CD-Rs, CD-RWs, MOs, DVDs, Blu-ray disks and portable hard disks. Further, a semiconductor memory such as a USB (Universal Serial Bus) memory is an example of a recording medium.
Alternatively, the program and the data may be provided (or downloaded) from the server to the information processing apparatus 3 through the network 4. For example, the program and the data may be provided to the information processing apparatus 3 through the communication IF 34. Further, the program and the data may be inputted to the information processing apparatus 3 from an input device described below which is connected to the peripheral IF 35.
The communication IF 34 is an example of a communication unit provided in the information processing apparatus 3, and is illustratively connected to the network 4 to enable communication through the network 4.
Upon focusing on a reception process, the communication IF 34 is an example of a receiver (which may be referred to as an "acquiring unit") which receives information transmitted from the vital sensor 2 to the information processing apparatus 3.
Meanwhile, upon focusing on a transmission process, the communication IF 34 is an example of a transmitter which transmits the control signal generated by the processor 31 to the vital sensor 2, the air conditioner 7 and the luminaire 8, for example. An Ethernet (registered trademark) card may be illustratively applied to the communication IF 34.

The communication IF 34 may be connected with the communication IF 25 of the vital sensor 2 without being routed through the network 4 to enable direct communication with the vital sensor 2.

The peripheral IF 35 is illustratively an interface which connects peripheral devices to the information processing apparatus 3.

The peripheral devices may include an input device which inputs information to the information processing apparatus 3, and an output device which outputs information generated by the information processing apparatus 3.

The input device may include a keyboard, a mouse and/or a touch panel. The output device may include a display and/or a printer.

By the way, when a heartbeat of the user is measured by using the wireless sensor 21 without contacting the user and when a physical motion (may be referred to as a "body motion") of the user becomes large to some extent, for example, it is difficult to detect a heartbeat component from the detected signal of the wireless sensor 21.

For example, during an exercise or an activity of the user, since a motion larger than a motion during rest would be occurred, a distance between the vital sensor 2 and a skin changes according to the body motion.

Illustratively, when the vital sensor 2 is put in a pocket of clothes of the user or when the vital sensor 2 is attached on the clothes of the user with the harness, the distance between the vital sensor 2 and the skin is easy to change according to the body motion.

Hence, a body motion derived signal component corresponding to the change in the distance is added as a noise component to the detected signal of the wireless sensor 21 in addition to a heartbeat derived signal component corresponding to the change in the distance. Hence, as the body motion of the user becomes larger, a noise component for a detection target of the heartbeat component is easily mixed in the detected signal of the wireless sensor 21, and therefore, it is difficult to detect the heartbeat component in the detected signal.

Not only in case of the wireless sensor 21 but also in case of a sensor such as an earclip which optically measures a change in a bloodstream, a bloodstream amount changes due to a body motion. Therefore, in addition to a heartbeat derived signal component, a body motion derived signal component is mixed as a noise component in a sensor detected signal.

Further, when the bloodstream amount changes due to the body motion, a blood pressure changes. Therefore, in a heartbeat sensor which uses a pressure sensor or a piezoelectric sensor, a body motion derived signal component becomes a noise component for a heartbeat derived signal component.

In a sensor such as an electrocardiograph or a phonocardiograph which measures a change in a potential of a heart muscle or a change in sound, since user's muscle moves due to the body motion, a body motion derived signal component becomes a noise component for a heartbeat derived signal component.

In short, independent to a measuring scheme of the heartbeat sensor, in other words, independent to a type of the heartbeat sensor, the signal component corresponding to the body motion is easily mixed as the noise component in a sensor detected signal when a body motion occurs during a user's exercise or activity.

By applying the above-described BPF to a detected signal of the wireless sensor 21, it may be possible to cancel a noise component which is not a heartbeat derived signal component to be detected.

In this regard, as illustrated in FIG. 5, for example, a time length (or a time interval) of a heartbeat per beat tends to vary according to a degree of a heart rate per unit time. In FIG. 5, a horizontal axis indicates a heart rate x(n) (n is a positive integer) at a given timing, and a vertical axis indicates the following heart rate x (n+1). Meanings of reference numerals 122, 124, 126 and "d" in FIG. 5 will be described below.

For example, as the heart rate per unit time increases, a variation of the time length per beat tends to decrease. Conversely, as the heart rate per unit time decreases, a variation of the time length per beat tends to decreases.

Illustratively, in an average frequency band (for a non-restrictive example, 0.8 to 4.0 Hz) in which a heartbeat component is likely to appear, a variation of ±20% possibly occurs in a time length per beat at 50 beats per unit time (e.g. one minute). Meanwhile, a variation of ±5% possibly occurs in a time length per beat at 120 beats higher than the 50 beats. Such a "variation" may be considered as "heartbeat characteristics" of the biological object.

Hence, when the filter characteristics of the BPF (e.g. passband width) is statically set to a specific heart rate such as 60 beats or 120 beats, a heartbeat component may lack or a cancelation of a noise component may be insufficient according to a degree of the heart rate. As a result, a detection accuracy of the heart rate may be decreased.

Hence, in the present embodiment, the filter characteristics of the BPF are adaptively changed according to a degree of the heart rate. When, for example, the heart rate per unit time is higher, the passband width of the BPF may be set to be narrower and, when the heart rate per unit time is lower, the passband width of the BPF may be set to be wider.

By making such variable bandwidth setting, it is possible to reduce a miss to receive a heartbeat component and enhance a cancellation rate of a noise component in an average frequency band (also referred to as a "heartbeat appearance band" for descriptive purposes) in which a heartbeat component is assumed to appear. The degree of the heart rate can be determined according to a degree of a frequency having a peak value in a result of the frequency analysis on the detected signal of the wireless sensor 21.

However, when a body motion of the user occurs, since a body motion derived signal component becomes large, a probability of erroneous detection becomes high on the ground that a peak value of the body motion derived signal component is easily detected as a peak value of a heartbeat component.

For example, as a change in a distance between the wireless sensor 21 and the user increases, an amplitude of the detected signal detected by the wireless sensor 21 tends to increase. Meanwhile, as a speed of the change in the distance increases, a frequency of the detected signal tends to increase.

Hence, a body motion derived signal component larger than the heartbeat component is easily to appear in the heartbeat appearance band in a result of the frequency analysis on the detected signal of the wireless sensor 21. As a result, a peak value of the body motion derived signal component, in other words, a peak value of a noise component for the heartbeat component to be detected would erroneously be detected as the heartbeat component.

When such erroneous detection occurs, the passband width of the BPF is set based on the frequency of the noise component. Hence, the heartbeat component lacks or a cancelation of the noise component may be insufficient. Therefore, a detection accuracy of the heart rate would decrease.

The decrease in the detection accuracy of the heart rate can be prevented or suppressed by, for example, estimating a heart rate of the user by using means different from the wireless sensor 21 to set and control the filter characteristics of the BPF based on the estimated heart rate.

An example of the different means available to estimate the heart rate of the user is, for example, to estimate the heart rate of the user based on a body motion of the user. The body motion of the user can be detected by the inertial sensor 22.

For example, a change corresponding to a magnitude and a speed of the body motion appears in a detected signal of the inertial sensor 22. As the magnitude of the body motion and the speed of the body motion increase, the exercise intensity of the user increases. Further, as the exercise intensity increase, the heart rate of the user tends to increase.

In this regard, a change in a heart rate (e.g. how the heart rate increases or decreases) associated with an exercise intensity of the user is an individual difference. For example, even in case of the same exercise intensity, the change in the heart rate differs per user. Hence, when a heart rate is estimated based on an exercise intensity, an error in an estimated heart rate is produced due to an individual difference.

When the filter characteristics of the BPF are set and controlled based on the estimated heart rate in which the error has been produced, naturally, a heartbeat component lacks or a noise component cannot be sufficiently canceled, and therefore a detection accuracy of a heart rate lowers.

Hence, in ,for example, a time domain in which an exercise intensity is less than a threshold value and the heart rate of the user is relatively stable, a change in a heart rate associated with the exercise intensity (in other words, a relation between the exercise intensity and the change in the heart rate) is actually measured based on a wireless sensor value and an inertial sensor value.

Further, the relation between the exercise intensity and the change in the heart rate in the time domain in which the exercise intensity of the same user is the threshold value or more is estimated based on the actually measured relation. The estimated relation is based on the relation actually measured from individual users, and therefore is made appropriate for the individual users. Consequently, it is possible to cover the individual difference in the change in the heart rate with respect to the exercise intensity.

By setting and controlling the filter characteristics of the BPF according to the estimated heart rate obtained based on the relation which is made appropriate per user, it is possible to prevent or suppress a decrease in a detection accuracy of a heart rate for each user in a time domain in which an exercise intensity is a threshold value or more, for example.

Consequently, even in a time domain in which a relatively many body motion derived noise component appears in a detected signal of the wireless sensor 21 not only during a rest time of the user but also during an exercise or an activity, it is possible to precisely measure the heart rate of the user. Since the measurement accuracy of the heart rate improves, it is also possible to improve, for example, an accuracy and efficiency to control a spatial environment in which the user is positioned.

In addition, a time domain in which an exercise intensity is less than the threshold value and a heart rate of the user is relatively stable will be referred to as a "low load area" for descriptive purposes. By contrast with this, a time domain in which an exercise intensity is the threshold value or more will be referred to as a "high load area" descriptive purposes.

FIG. 6 illustrates an example of temporal changes in a heart rate (an actual measured value and an estimated value) of a given user and a METs value (described below) which is an index of an exercise intensity. In FIG. 6, symbol A indicates an example of a temporal change in a METs value, and symbol B indicates an example of a temporal change in a heart rate (actual measured value) of the same user detected based on a wireless sensor value. Further, symbol C indicates an example of a temporal change in a heart rate (estimated value) of the user estimated based on the METs value.

The "high load area" of a non-restrictive example may correspond to a time domain in which the METs value is a threshold value (e.g. 4) or more as illustrated in FIG. 6. A heart rate of the "high load area" is estimated, and therefore the "high load area" may be paraphrased as a "heartbeat estimation area".

(Operation example)

Some operation examples of the sensor system 1 according to the present embodiment will be described. In an example described below, a wireless sensor value and an inertial sensor value of the vital sensor 2 are processed by the information processing apparatus 3. However, the same process described below may be performed by the vital sensor 2 (e.g. processor 23).

(First example)

FIG. 7 is a flowchart illustrating an operation example of the sensor system 1 according to a first example. As illustrated in FIG. 7, the information processing apparatus 3 receives a wireless sensor value and an inertial sensor value from a sensor unit 2 (processes P11 and P21).

For example, a processor 31 of the information processing apparatus 3 calculates an exercise intensity of a user based on the inertial sensor value in response to reception of the inertial sensor value (process P22). FIG. 8 illustrates an example of an exercise intensity calculation process.

As illustrated in FIG. 8, the processor 31 may calculate the number of steps (which may be referred to as a "walking cadence") of the user per unit time (for example, during one second) based on an inertial sensor value (process P221). Similar to general pedometers, the number of steps can be obtained by, for example, counting in the processor 31 the number of times that the inertial sensor value exceeds a threshold value.

The processor 31 may calculate a walking speed based on the walking cadence according to the calculated walking cadence (process P222).

In this regard, the walking cadence and the walking speed can be linked by a relational equation. The relational equation may be derived by performing a curve-fitting on a plurality of actual measured values.

A non-restrictive example of the relational equation of the walking cadence and the walking speed can be expressed by following polynominal equation (1) as illustrated in FIG. 9.

$$y=1E-05x^3-0.0014x^2+0.0725x-0.0119 \ldots (1)$$

In FIG. 9, a horizontal axis "x" indicates a walking cadence [the number of steps/second], and a vertical axis "y" indicates a walking speed [km/h] per unit time (illustratively, one hour). Further, "1E-05" represents "$1x10^{-5}$".

As a walking distance per unit time increases, a step width of a distance per step and the number of steps also tend to increase. For example, a step width = height x 0.37 is applicable when the user walks 70 m per minute (= 4.2 km per hour), a step width = height x 0.45 is applicable when the user walks 90 m per minute (= 5.4 km per hour) and a step width = height x 0.5 is applicable when the user walks 110 m per minute (= 6.6 km per hour). Therefore, it is possible to approximately calculate the walking speed corresponding to a walking distance per unit time based on the above step width and walking cadence.

The processor 31 may calculate the walking speed by calculating equation (1) based on the walking cadence calculated in process P221 in FIG. 8.

In addition, the relation illustrated in FIG. 9 may be expressed by, for example, table data and be stored in a storage unit 30 of the information processing apparatus 3 (see FIG. 4). The processor 31 may determine a walking speed rate for the walking cadence with reference to the table data without performing arithmetic processing.

The processor 31 may calculate an exercise intensity of the user based on the walking speed according to the calculated walking speed (process P223 in FIG. 8). Information of the calculated exercise intensity may be associated with information of a heart rate detected based on the wireless sensor value in heart rate calculation process P14 described below, and be stored in the storage unit 30.

The processor 31 may estimate a relation between the exercise intensity and a change in the heart rate in the high load area based on the pieces of information of the exercise intensity and the heart rate in the low load area stored in the storage unit 30, and generate information 100 indicating this relation. The information 100 may be referred to as the "exercise intensity-to-heart rate information 100". A generation example of the exercise intensity-to-heart rate information 100 will be described below.

In addition, the processor 31 may store an information set of an exercise intensity and a heart rate in the storage unit 30 irrespectively of whether a calculated exercise intensity is high or low, or may store this information set in the storage unit 30 only when the calculated exercise intensity is less than the threshold value (in other words, the low load area).

The exercise intensity is an index value indicative of an activity amount of a person, and may be expressed by a METs value. METs is an abbreviation of "Metabolic equivalents".

The METs value may be a numerical value which expresses a relative value (e.g. a multiple number) of a metabolic rate (or a calorie consumption amount) during an activity of a person with respect to a metabolic rate during rest. A table in which METs values are associated with each activity of a person is referred to as a "METs table". The METs table is published by the National Institute of Health and Nutrition, for example.

FIG. 10 illustrates an example of a relation between walking speeds and METs values. A walking speed = 0 [km/h] is associated with a METs value = 1 and corresponds to a reference exercise intensity during rest. As illustrated in FIG. 10, as the walking speed increases, the METs value also increases.

For example, in case of a walking speed = 2.5 [km/h], the METs value is three times as a reference METs value (=1). In case of a walking speed = 4 [km/h], the METs value is five times as the reference METs value.

When the METs value is determined, a current heart rate can be determined based on an age of the user and the heart rate during rest, for example. The METs value can be expressed by following equation (2), for example.

$$\text{METs value} = (\text{heart rate} - \text{heart rate during rest}) / (\text{maximum heart rate} - \text{heart rate during rest}) \times 10 \quad \ldots \quad (2)$$

The "maximum heart rate" in equation (2) can be calculated as a simplified equation of "220 - age".

Consequently, the processor 31 can calculate the current heart rate based on the METs value according to equation (2) (process P23 in FIG. 7).

As illustrated in, for example, FIG. 11, in the low load area in which the exercise intensity is less than the threshold value (NO in process P231), the processor 31 may calculate an estimated heart rate according to equation (2) based on the exercise intensity calculated in process P22 in FIG. 7 (process P223 in FIG. 8).

Meanwhile, in the high load area in which an exercise intensity is the threshold value or more (YES in process P231), the processor 31 may calculate the estimated heart rate based on the exercise intensity-to-heart rate information 100 stored in the storage unit 30 (process P232).

When the estimated heart rate is calculated, the processor 31 may determine filter characteristics of a BPF applied to a wireless sensor value and matching the estimated heart rate as illustrated in FIG. 7 (process P24). Hereinafter, the filter characteristics of the BPF may be abbreviated as "BPF characteristics".

The processor 31 may convert the estimated heart rate into a frequency and set the converted frequency to a center frequency of the BPF to be applied to the wireless sensor value, for example. The heart rate can be converted into the frequency by dividing the heart rate by 60 (seconds), for example. Further, the processor 31 may set a narrower bandwidth to the BPF when the estimated heart rate is higher. A detailed determination example of the BPF characteristics will be described below.

In this regard, an estimated timing (or an estimated period) of a heart rate and an appearance timing (or an appearance period) of a heartbeat component appearing in a wireless sensor value mismatch in some cases. This mismatch remarkably occurs when the user starts an exercise or an activity.

For example, a timing at which the heart rate of the user starts increasing according to an exercise intensity tends to delay from a timing at which the exercise intensity reaches a predetermined intensity after the user starts the activity or the exercise.

Hence, even when the BPF is applied to the wireless sensor value at the estimated timing of the heart rate, the above timing mismatch temporarily lowers a detection accuracy of a heartbeat component or a cancellation capability for a noise component.

Hence, as indicated by a dotted line in FIG. 7, for example, the processor 31 may delay a timing to apply the BPF to the wireless sensor value, from the estimated timing of the heart rate (process P25). A delay time of a non-restrictive example may be statically or dynamically set to a range of several seconds to several tens of seconds. When the delay time is dynamically set, and when, for example, the inertial sensor value indicates that the exercise intensity changes from a high state to a low state, a timing delay time may be set to shorten gradually or stepwise.

In addition, a delay process may be performed in one of processes before BPF characteristics determination process P24. For example, the processor 31 may delay an exercise intensity calculation process or a heart rate estimation process, or may delay a process of determining BPF characteristics based on an estimated heart rate. Alternatively, by delaying a plurality of processed in a distributed manner, a delay time equal to the delay time in delay process P25 may be realized.

By applying the BPF of the BPF characteristics determined in process P24 to the wireless sensor value received in process P11 at a delayed timing, the processor 31 may filter the wireless sensor value (process P12). According to the filtering, the body motion derived noise component is removed from the wireless sensor value.

The processor 31 may detect a heartbeat component indicative of a distinctive change corresponding to a heartbeat as a "feature point" from the filtered wireless sensor value (process P13). The "feature point" may be, for example, a point at which a first derivation becomes zero in a signal waveform of the filtered wireless sensor value.

In response to detection of the "feature point", the processor 31 can calculate a heart rate per minute by calculating a time interval (e.g. "second") at the feature point and dividing one minute (= 60 seconds) by the calculated time interval (process P14).

The information of the calculated heart rate may be stored in the storage unit 30 together with the information of the exercise intensity calculated in process P22 (process P223 in FIG. 8) in FIG. 7 as described above.

Further, the information of the calculated heart rate may be used as a parameter to control a spatial environment in which the user of the vital sensor 2 is positioned (process P15). Furthermore, the information of the calculated heart rate may be optionally outputted to an output device such as a display or a printer as indicated by the dotted line in FIG. 7 (process P16).

In addition, information of the walking cadence, the walking speed and the exercise intensity obtained in process P22, information of the estimated heart rate obtained in process P23, information of the BPF characteristics determined in process P24 and information of the characteristic point detected in process P13 may be optionally outputted to the output device such as the display or the printer.

In this case, the output device can check a calculation status of a walking cadence, a walking speed, an exercise intensity or an estimated heart rate or check whether or not a setting situation or a setting of the BPF characteristics is appropriate.

(Generation example of exercise intensity-to-heart rate information)

Next, the generation example of the exercise intensity-to-heart rate information 100 will be described with reference to FIGS. 12 to 20.

As illustrated in FIG. 12, the information processing apparatus 3 (e.g. the processor 31) may generate an approximate equation of a change in a heart rate with respect to an exercise intensity in the high load area based on the information set of the exercise intensity and the heart rate in the low load area stored in the storage unit 30 (process P31). In addition, the high load area of a non-restrictive example is assumed to be an area whose METs value is 4.0 or more.

Based on, for example, a general rest-time heart rate with respect to an age of the user, and a maximum heart rate (e.g. 220 - age), it is possible to calculate an estimated heart rate per general exercise intensity with respect to an age (such an estimated heart rate may be referred to as an "age estimated heart rate" for descriptive purposes) according to above equation (2).

The "age estimated heart rate" of a non-restrictive example calculated by using equation (2) is indicated in the second column from the right in FIG. 15.

FIG. 15 illustrates age estimated heart rates calculated according to equation (2) in case where METs values (walking speed) are 1.0 (0 km/h), 1.5 (1.5 km/h), 3.0 (2.5 km/h), 3.3 (3.0 km/h) and 3.8 (3.5 km/h).

For example, when the METs value (walking speed) is 1.0 (0 km/h), the age estimated heart rate is 61.3 [bpm] and, when the METs value (walking speed) is 1.5 (1.5 km/h), the age estimated heart rate is 74 [bpm].

Further, for example, when the METs value (walking speed) is 3.0 (2.5 km/h), the age estimated heart rate is 84 [bpm] and, when the METs value (walking speed) is 3.3 (3.0 km/h), the age estimated heart rate is 94.19 [bpm]. When the METs value (walking speed) is 3.8 (km/h), the age estimated heart rate is 107 [bpm].

By performing a curve-fitting on the above five pieces of data as illustrated in FIG. 13, a relation between an exercise intensity (METs value) and an age estimated heart rate can be expressed by a polynomial as in following equation (3).

$$y=3.4851x^2+0.7088x+62.128 \ldots (3)$$

In addition "x" represents an exercise intensity (METs value), and "y" represents an age estimated heart rate.

By contrast with this, the heart rates of the user with respect to the five exercise intensities are assumed to be measured as illustrated in a right end column in FIG. 15. In addition, the heart rate illustratively corresponds to a heart rate calculated based on a wireless sensor value in process P14 in FIG. 7. The heart rate may be referred to as a "measured heart rate" or an "actual measured heart rate" for descriptive purposes in comparison with the above "age estimated heart rate".

In the example in FIG. 15, when the METs value (walking speed) is 1.0 (0 km/h), the measured heart rate is 65 [bpm] and, when the METs value (walking speed) is 1.5 (1.5 km/h), the measured heart rate is 85 [bpm].

Further, when the METs value (walking speed) is 3.0 (2.5 km/h), the measured heart rate is 102 [bpm] and, when the METs value (walking speed) is 3.3 (3.0 km/h), the measured heart rate is 111 [bpm]. When the METs value (walking speed) is 3.8 (km/h), the measured heart rate is 132 [bpm].

By performing a curve-fitting on the above five pieces of data as illustrated in FIG. 14 similar to the "age estimated heart rate", a relation between an exercise intensity (METs value) and a "measured heart rate" can be expressed by a polynomial as in following equation (4).

$$y=2.4314x^2+9.0462x+65.099 \ldots (4)$$

In addition "x" represents an exercise intensity (METs value), and "y" represents a "measured heart rate".

FIG. 18 illustrates an example of a relation (polynomial) between an exercise intensity (illustratively, a walking speed) and a measured heart rate in the low load area obtained from three subjects A to C similar to the example in FIG. 14. FIG. 18 illustrates an example where polynomials (5A) to (5C) are obtained for the subjects A to C.

$$y=3.677x^2+0.0446x+61.395 \ldots (5A)$$

$$y=3.949x^2+3.3961x+65.966 \ldots (5B)$$

$$y=3.5099x^2-0.7961x+58.061 \ldots (5C)$$

In addition "x" represents an exercise intensity (METs value), and "y" represents a "measured heart rate".

The information processing apparatus 3 (e.g. processor 31) can generate an approximate equation of a change in a heart rate with respect to an exercise intensity in the high load area (e.g. an area in which a METs value exceeds 4.0) based on the relation illustrated in FIG. 14 and equation (4), for example.

Illustratively, the processor 31 may calculate a maximum heart rate which is a constant S per exercise intensity according to following equation (6).

S = (10 ÷ exercise intensity) × (measured heart rate - rest-time heart rate) + heart rate during rest time ... (6)

When the "rest-time heart rate" is assumed to be "47", the constant S per exercise intensity in the low load area is calculated as a value illustrated in a right end column in FIG. 16A according to equation (6).

Further, the processor 31 may generate an approximate equation (illustratively, a polynomial) which interpolates an interval in which data lacks, based on a measured heart rate per exercise intensity in the low load area and a maximum value of an age estimated heart rate per exercise intensity, and calculate the constant S in the high load area.

FIG. 17 illustrates an example of the exercise intensity and the constant S (polynomial). FIG. 17 illustrates an example where six pieces of data of the five pieces of the constant S obtained in the low load area and data of a maximum value of the age estimated heart rate (e. g. 162.6 [bpm]) in case where an exercise intensity is 8 METs are curve-fitted.

In this regard, in the example in FIG. 17, the maximum value of the age estimated heart rate (e.g. 162.6 [bpm]) in case where the exercise intensity is 8 METs is regarded as a "measured heart rate" at this exercise intensity for descriptive purposes. In the example in FIG. 17, a relation between the exercise intensity (METs value) and the constant S can be expressed by a polynomial as in following equation (7).

$$y=-1.8222x^2+7.4198x+248.84 \ldots (7)$$

In addition "x" represents an exercise intensity (METs value), and "y" represents the "constant S".

The processor 31 can illustratively calculate the estimated heart rate per exercise intensity in the high load area in which the exercise intensity exceeds 4 METs as illustrated in a right end column in FIG. 16B according to the relation in equation (7).

For example, when the METs value (walking speed) is 5.0 (4.0 km/h), the estimated heart rate can be calculated as 143.692 [bpm] and, when the METs value (walking speed) is 6.3 (4.5 km/h), the estimated heart rate can be calculated as 158.0448219 [bpm].

Further, when the METs value (walking speed) is 7.0 (5.0 km/h), the estimated heart rate can be calculated as 162.14356 [bpm].

In addition, in FIG. 16B, the "estimated heart rate" per exercise intensity in the low load area may be regarded to be equal to the "measured heart rate" per exercise intensity for descriptive purposes.

By performing a curve-fitting on the estimated heart rate per exercise intensity calculated from the high load area, for example, it is possible to find a relation between the exercise intensity (METs value) and the "estimated heart rate" in the high load area.

FIG. 19 illustrates a non-restrictive example of a relation between an exercise intensity and a measured heart rate in the high load area obtained based on a relation between an exercise intensity and a measured heart rate in the low load area of each of the three subjects A to C illustrated in FIG. 18.

FIG. 19 illustrates an example where relations expressed by following polynomials (7A) to (7C) are obtained as an example of relations between an exercise intensity (walking speed) and an estimated heart rate in the high load area of the subjects A to C.

$$y=-1.2204x^2+23.499x+48.425 \ldots (7A)$$

$$y=-0.8038x^2+16.853x+83.583 \ldots (7B)$$

$$y=-0.2161x^2+11.477x+66.754 \ldots (7C)$$

In addition, "x" represents an exercise intensity (METs value), and "y" represents an "estimated heart rate".

As illustrated in FIG. 20, a relation between an exercise intensity and an estimated heart rate across the low load area and the high load area can be found as a relation obtained by synthesizing the polynomial expressing the relation in the low load area illustrated in FIG. 18 and the polynomial expressing the relation in the high load area illustrated in FIG. 19.

The information processing apparatus 3 (e.g. processor 31) may generate a function illustrated in FIG. 20 as an approximate equation in process P31 in FIG. 12. The processor 31 may generate the exercise intensity-to-heart rate information 100 based on the approximate equation to store in the storage unit 30 (process P32). In addition, the exercise intensity-to-heart rate information 100 may be an approximate equation itself expressing the relation illustrated in FIG. 20.

The exercise intensity-to-heart rate information 100 is used in above-described heart rate estimation process P23 in FIG. 7. In addition, when the exercise intensity-to-heart rate information 100 of the low load area to the high load area is obtained, the processor 31 does not need to perform above-described determination process P231 in FIG. 11.

For example, the processor 31 may determine an estimated heart rate based on the exercise intensity-to-heart rate information 100 without determining which one of the low load area and the high load area the exercise intensity calculated in process P22 in FIG. 7 belongs.

In other words, the exercise intensity-to-heart rate information 100 maybe found from the low load area and the high load area or may be found from the high load area. In case of the latter, the processor 31 may regard a measured heart rate in the low load area as an estimated heart rate in the low load area and determine BPF characteristics in process P24 in FIG. 7.

As described above, according to the first embodiment, a heart rate in case where an exercise intensity is the threshold value or more (high load area) is estimated based on a relation between an exercise intensity and a heart rate obtained based on a wireless sensor value while the exercise intensity obtained based on an inertial sensor value is less than the threshold value (low load area).

In other words, a relation between an exercise and a change in a heart rate while the exercise intensity of the same user is the threshold value or more is estimated based on a relation between the exercise intensity and the change in the heart rate measured while the exercise intensity of the same user is less than the threshold value.

Consequently, the relation between the exercise intensity and the change in the heart rate in the high load area in which the exercise intensity is the threshold value or more can be made appropriate for individual users, and an individual difference in the change in the heart rate with respect to the exercise intensity can be covered. Consequently, it is possible to improve a detection accuracy of a heartbeat signal of the heartbeat sensor 21 for each individual.

Further, by setting and controlling the filter characteristics of the BPF according to the estimated heart rate obtained based on the relation which is made appropriate per user, it is possible to prevent or suppress a decrease in a detection accuracy of a heart rate for each user in a time domain in which an exercise intensity is a threshold value or more, for example.

Consequently, even in a time domain in which a relatively many body motion derived noise component appears in a detected signal of the wireless sensor 21 not only during a rest time of the user but also during an exercise or an activity, it is possible to precisely measure the heart rate of the user. Since the measurement accuracy of heart rate improves, it is also possible to improve, for example, an accuracy and efficiency to control a spatial environment in which the user is positioned.

Further, by controlling the BPF characteristics applied to a wireless sensor value according to a frequency associated with a heart rate estimated based on an inertial sensor value, it is possible to efficiently cancel the noise component appearing in a heartbeat appearance band.

Accordingly, it is possible to improve a detection accuracy of a heartbeat signal based on the wireless sensor value. For example, it is possible to improve a detection accuracy of a heartbeat signal in a band of 1.5 to 4.0 Hz which is considered as being easily influenced by a body motion derived noise component within a band of 0.8 to 4.0 Hz which is an example of the heartbeat appearance band.

Further, by delaying a timing to apply the BPF to a wireless sensor value from an estimated timing of a heart rate estimated based on an inertial sensor value, it is possible to reduce a miss to receive a heartbeat component even when the heartbeat component appears late in the wireless sensor value in response to a change in the inertial sensor value.

(Modified example 1)

In addition, BPF bandwidth control based on an estimated heart rate does not need to be indispensable control. By, for example, controlling a center frequency of a BPF according to an estimated heart rate without performing BPF bandwidth control, it is possible to expect improvement in a measurement accuracy of heart rate.

(Modified example 2)

Further, a relation between an exercise intensity and a heart rate has been measured or estimated in the above first embodiment. However, the exercise intensity is correlated with an inertial sensor value, and therefore may be replaced with the inertial senor value or information such as a body motion amount, a walking cadence or a walking speed obtained from the inertial sensor value.

For example, a relation measured in a low load area and a relation estimated in a high load area may be a relation between an inertial sensor value and a heart rate or a relation between a body motion amount, a walking cadence or a walking speed obtained from the inertial sensor value, and a heart rate.

In other words, in the relations illustrated in FIGS. 13, 14 and 18 to 20, the horizontal axes may indicate an inertial sensor value or information such as a body motion amount, a walking cadence or a walking speed obtained from the inertial sensor value.

One or both of above modified examples 1 and 2 also apply to a following second embodiment.

(Second example)

In the first example, the BPF characteristics are determined based on an estimated heart rate without depending on a degree of a heart rate estimated based on an inertial sensor value. However, the BPF characteristics may be determined based on a wireless sensor value without depending on the inertial sensor value in accordance with an estimated heart rate.

When, for example, a heart rate estimated based on an inertial sensor value is high, a METs value which is an index of an associated exercise intensity also tends to be high. Hence, when the estimated heart rate (or METs value) is the threshold value or more (in other words, in the high load area), the BPF characteristics may be determined based on the heart rate estimated based on the inertial sensor value as in the first embodiment.

When the estimated heart rate (or METs value) is higher, it is said that a body motion derived noise component is likely to be mixed in a heartbeat appearance band of the wireless sensor value.

By contrast with this, when the heart rate estimated based on the inertial sensor value is less than the threshold value (in other words, the low load area), even when the BPF characteristics are determined based on the wireless sensor value, it is possible to sufficiently cancel the body motion derived noise component.

In other words, when the heart rate (or the METs) value estimated based on the inertial sensor value is less than the threshold value, it may be considered that an impact on detection of a heartbeat component caused by the body motion derived noise component would be a little.

Hence, in the second example, as illustrated in FIG. 21, which one of the inertial sensor value and the wireless sensor value is to be used for determining the BPF characteristics may be controlled according to a determination with threshold value (process P23a) for the estimated heart rate (or the METs value).

When, for example, the estimated heart rate (or METs value) is the threshold value or more (YES in process P23a), a processor 31 of an information processing apparatus 3 may determine the BPF characteristics similar to the first embodiment (process P24). For example, the processor 31 may determine the BPF characteristics based on a frequency associated with a heart rate estimated based on exercise intensity-to-heart rate information 100. The frequency corresponding to the heart rate may be referred to as a "heartbeat frequency" for descriptive purposes.

In addition, in the second embodiment, the processor 31 may also delay a timing to apply a BPF of the BPF characteristics determined in process P24 to a wireless sensor value similar to the first embodiment (process P25).

Meanwhile, when the estimated heart rate (or METs value) is less than the threshold value (NO in process P23a), the processor 31 may detect the heart rate based on the wireless sensor value, and determine the BPF characteristics based on a detected heartbeat frequency (processes P41 and P42).

A frequency analysis (process P41) may be used to detect the heart rate from the wireless sensor value. A fast Fourier transform (FFT) or a discrete Fourier transform (DFT) is applicable to the frequency analysis.

The wireless sensor value is converted from a time domain signal into a frequency domain signal (which may be referred to as a "frequency signal" for descriptive purposes) by the FFT or the DFT.

The processor 31 may detect a frequency having a peak value (such frequency may be referred to as a "peak frequency") in the frequency signal of the wireless sensor value as a "feature point" described above, for example. The peak frequency is an example of a frequency component indicative of a distinctive change corresponding to a heartbeat.

The processor 31 may determine the BPF characteristics based on the detected peak frequency (process P42). For example, the processor 31 may determine BPF characteristics having the peak frequency at the center frequency and a narrower bandwidth when the peak frequency is higher (or a wider bandwidth when the peak frequency is lower) as the BPF characteristics to be applied to the wireless sensor value.

In addition, the wireless sensor value received by the information processing apparatus 3 in process P11 may be stored in a storage unit 30 in preparation for the above frequency analysis process (P41). The wireless sensor value stored in the storage unit 30 is illustratively read by the processor 31 in process P12a and is applied with a BPF having the BPF characteristics determined in process P24 or P42.

A feature point detection process (P13), a heart rate calculation process (P14), a spatial environment control (P15) and a determination result output (P16) performed by the processor 31 after process 12a may be the same as those in the first example.

As described above, according to the second example, the same function and effect as those in the first example are obtained. Additionally, the BPF characteristics are controlled based on the wireless sensor value when the inertial sensor value indicates that an impact on detection of a heartbeat component due to a body motion derived noise component may be considered as being a little.

Accordingly, it is possible to improve a detection accuracy of a heartbeat signal in a band of 0.8 to 1.5 Hz in which the impact due to the body motion derived noise component may be considered as being a little within a band of 0.8 to 4.0 Hz which is an example of the heartbeat appearance band.

Delay process P25 in FIG. 21 may be performed before determination process P24 of the BPF characteristics. In other words, a process to determine the BPF characteristics based on an estimated heart rate may be delayed.

(Determination example of BPF characteristics)

Next, a detailed example of a process (e.g. process P24 in FIGS. 7 and 21 and process P12a in FIG. 21) to determine (or set) the above-described BPF characteristics will be described with reference to FIGS. 5 and 22 to 31.

A determination example of the BPF characteristics described below may be common to the aforementioned first and second examples. However, in the following description, a "reference heart rate" corresponds to a heart rate estimated based on the inertial sensor value in the first example, and corresponds to a heart rate estimated based on the inertial sensor value or the wireless sensor value in the second example.

For example, in the second example, the "reference heart rate" when YES is determined in threshold determination process P23a in FIG. 21 corresponds to the heart rate estimated based on the inertial sensor value. Meanwhile, the "reference heart rate" when NO is determined in process P23a corresponds to the heart rate detected based on the wireless sensor value.

As described above, FIG. 5 illustrates a relation between a heart rate per unit time and a variation of a time length per heartbeat. Reference numeral 122 denotes measured data (x(n), x(n+1)) of the heart rate and a straight line 124 indicates a median value of each measured data 122.

The straight line indicating the median value 124 is illustratively a straight line of each measured data 122 calculated according to a least-squares method, for example, and is assumed to be expressed as ax (n) + bx(n+1) + c = 0. In this regard, coefficients a, b and c are actual numbers.

Here, a length d of a perpendicular line drawn from each measured data 122 (x(n), x(n+1)) to the straight line 124 can be expressed by following equation (8).

$$d = \frac{|ax(n)+bx(n+1)+c|}{\sqrt{a^2+b^2}} \quad \ldots (8)$$

A measured point having a maximum value of the length d is assumed to be a maximum distance point 126 expressed by a coordinate (x(m), x(m+1)). The "m" represents an positive integer which satisfies $m \leq n$.

A straight line which passes through the maximum distance point 126 and a coordinate (a coordinate (120,114) in the example in FIG. 5) corresponding to "-0.1 Hz" in case of heartbeat frequency = 2 Hz represents a lower limit of a bandwidth, for example.

The straight line indicative of an upper limit of the bandwidth corresponds to a straight line which passes through a point (120, 126) and a point having the same width as that of the lower limit of a bandwidth at any of frequencies, for example. A bandwidth in case of heartbeat frequency = 2 Hz may be determined with reference to a maximum value and a minimum value of a next heartbeat at heart rate x(n) = 120 of the measured data 122 in FIG. 5.

Next, FIG. 22 is a diagram illustrating a setting example of the BPF characteristics. FIG. 22 illustrates an example of an upper limit and a lower limit of a band of the BPF calculated by substituting the reference heart rate in a preset equation.

In FIG. 22, a horizontal axis indicates a reference heart rate [Hz], and a vertical axis indicates a heart rate [Hz] corresponding to an upper limit and a lower limit of a bandwidth. In FIG. 21, the upper limit of the bandwidth is indicated by a band upper limit value 132, and the lower limit of the bandwidth is indicated by a band lower limit value 134. FIG. 22 illustrates the upper limit values and the lower limit values of the bandwidth at some reference heart rate.

The above "preset equation" may be determined by interpolation or extrapolation based on, for example, the maximum distance point 126 and reference coordinates (120, 126) and (120, 114) determined based on the measured data 122 illustrated in FIG. 5.

Next, FIG. 23 is a diagram illustrating an example of a passband of the BPF. In FIG. 23, a horizontal axis indicates a center frequency of the BPF, and a vertical axis indicates a bandwidth of the BPF. Further, in FIG. 23, a fixed minimum heartbeat 141 or a variable minimum heartbeat 144 indicates a heart rate corresponding to a lower limit of a band when a bandwidth corresponding to a given center frequency is adopted.

Furthermore, in FIG. 23, a fixed maximum heartbeat 142 or a variable maximum heartbeat 145 indicates a heart rate corresponding to a lower limit of a band when a bandwidth corresponding to a given center frequency is adopted.

The fixed minimum heartbeat 141 and the fixed maximum heartbeat 142 are comparative examples in case where a bandwidth does not change according to a heart rate in comparison with a case where the bandwidth of the BPF is variable in the present embodiment.

According to the variable minimum heartbeat 144 and the variable maximum heartbeat 145, the bandwidth of the BPF corresponds to a bandwidth indicated by reference numeral 148 near heart rate = 1 Hz and corresponds to a bandwidth indicated by reference numeral 149 which is narrower than the bandwidth 148 near heart rate = 4 Hz, for example. Thus, the wider bandwidth 148 is set at a lower heart rate, and the narrower bandwidth 149 is set at a higher heart rate.

FIG. 24 is a diagram illustrating a setting example of the BPF bandwidth. In FIG. 24, a horizontal axis indicates a center frequency, and a vertical axis indicates a bandwidth. In FIG. 24, a straight line 152 indicates an example of a setting value of a bandwidth with respect to the center frequency. For example, in examples described with reference to FIGS. 5 and 23, the bandwidth 152 may be set to decrease linearly in response to an increase of the center frequency.

A relation between the center frequency and the bandwidth may be enough to have a relation in which the bandwidth becomes wider when the center frequency becomes lower, and does not need to be a relation expressed by a straight line. For example, as indicated by reference numeral 154 in FIG. 24, the bandwidth may be set such that the bandwidth changes stepwise with respect to the center frequency. The relation between the bandwidth and the center frequency may be determined by interpolation and extrapolation using one or more suitable functions of a monotonic increase based on given two or more points determined based on an actual measured value, for example.

Next, FIGS. 25 to 28 are diagrams illustrating other setting examples of bandwidths. FIGS. 25 and 26 are diagrams illustrating examples of a heart rate distribution, and FIGS. 27 and 28 are diagrams illustrating examples of a heart rate statistical process. In FIGS. 25 and 26, horizontal axes indicate a time indicative of a difference between a heartbeat interval at a given point of time and a next heartbeat interval, and vertical axes indicate the number of measured times.

FIG. 25 illustrates a heart rate distribution whose heart rate is near 55 to 60, and FIG. 26 illustrates a heart rate distribution whose heart rate is near 75 to 80. In comparison between FIGS. 25 and 26, pieces of measured data concentrate more near the vertical axis at the heart rate near 55 to 60 than at the heart rate near 75 to 80, and the heart rate varies less. The measured data used for a statistical process may be data measured by an electrocardiograph which measures a heartbeat by placing an electrode in contact with a biological object, for example.

FIGS. 27 and 28 illustrate examples of the statistical process of actual measured data at the heart rate near 60 and at the heart rate near 120. In examples of the statistical process illustrated in FIGS. 27 and 28, a probability distribution of 532 pieces of data is calculated, and the bandwidth of the BPF is calculated such that the measured data is in a range of a certain probability or more.

In the example of the statistical process in FIG. 27, by determining a range of 8 $\sigma$ of the calculated probability distribution and CP value = 1.33 at the heart rate near 60, the bandwidth is $\pm$0.165 Hz. Here, $\sigma$ represents a standard deviation, and "CP" represents "Process Capability". The same bandwidth as this bandwidth at the heart rate near 120 is provided in case of CP value = 2.16.

Similarly, in the example of the statistical process in FIG. 28, by determining a range of 8 $\sigma$ of the calculated probability distribution and CP value = 1.33 at the heart rate near 120, the bandwidth is $\pm$0.1015 Hz. The same bandwidth as this bandwidth at the heart rate near 60 is provided in case of CP value = 0.82.

The statistical process of an actual measured value is performed as described above, and the bandwidth of the BPF which enables data detection at a given probability or more is set. In this case, the relation between the bandwidth and the center frequency corresponding to the reference heart rate may be linearly determined based on given two points or may be determined by interpolation by performing the above statistical process on the center frequency of a shorter interval.

Alternatively, the relation between the bandwidth and the center frequency may be determined by interpolation and extrapolation by using one or more suitable functions of a monotonic increase based on each point when the given two or more points are determined, or may be set such that the bandwidth changes stepwise with respect to the center frequency.

FIGS. 29 and 30 are diagrams illustrating setting examples of a bandwidth. In FIGS. 29 and 30, horizontal axes indicate a frequency [Hz], and vertical axes indicate a gain [dB]. In FIGS. 29 and 30, a heartbeat appearance band 162 is 0.8 to 4.0 Hz, for example.

According to a frequency analysis result (e.g. FFT result) 160 of a wireless sensor value illustrated in FIG. 29, a peak gain indicated by reference numeral 163 exists in the heartbeat appearance band 162. A frequency corresponding to the peak gain 163 is set to the center frequency of the BPF, and a bandwidth indicated by reference numeral 164 is set to the bandwidth of the BPF, for example. Meanwhile, according to an FFT result 165 illustrated in FIG. 30, a peak gain indicated by reference numeral 168 exists in the heartbeat appearance band 162. The frequency corresponding to the peak gain 168 is set to the center frequency of the BPF.

Here, since the center frequency corresponding to the peak gain 168 is higher than the frequency corresponding to the peak gain 163 in FIG. 29, a bandwidth 166 narrower than the bandwidth 164 in FIG. 29 is set to the bandwidth of the BPF.

In this way, the center frequency of the BPF is set to the frequency corresponding to the reference heart rate, and the bandwidth is adaptively controlled according to a degree of the center frequency. Therefore, it is possible to efficiently remove a noise component which appears in the heartbeat appearance band.

In other words, by using the BPF which has the variable center frequency and variable bandwidth depending on heartbeat characteristics of a biological object, it is possible to efficiently remove an unnecessary signal component in the heartbeat appearance band without depending on a degree of the heart rate. Accordingly, it is possible to improve a detection accuracy of a heartbeat signal in the heartbeat appearance band.

FIG. 31 is a diagram illustrating an example of bandwidth information. Bandwidth information 170 illustrated in FIG. 31 may be stored in the storage unit 30 of the information processing apparatus 3, for example. As a non-restrictive example, the bandwidth information 170 may include reference heart rate information 171, BPF width lower limit information 172 and BPF width upper limit information 173.

The processor 31 of the information processing apparatus 3 is available to determine and set a band of the BPF corresponding to the reference heart rate with reference to the bandwidth information 170.

(Others)

In the above-described embodiments including the various examples, a wireless sensor such as a Doppler sensor is used as an example of a heartbeat sensor 21. However, the heartbeat sensor 21 may be a sensor such as an earclip which optically measures a change in a bloodstream, for example.

Even when a bloodstream amount changes due to a body motion, a body motion derived signal component is mixed as a noise component in a sensor detected signal, it is possible to efficiently remove the noise component by controlling the above-described variable BPF characteristics.

In case of the heartbeat sensor 21 which optically measures the change in the flood flow, even when a noise component is mixed in a sensor detected signal on the ground that external light is mixed in reflected light from the bloodstream in outdoor, the noise component can be removed by controlling the above-described variable BPF characteristics.

Further, the heartbeat sensor 21 may be an electrocardiograph or a phonocardiograph which measures a change in a potential of a heart muscle or a change in sound. Even when a body motion derived signal component is mixed as a noise component in a sensor detected signal on the ground that a user's muscle moves due to a body motion, it is possible to efficiently remove the noise component by controlling the above-described variable BPF characteristics.

## Claims

**1.** A sensor information processing apparatus comprising:

a receiver (34) configured to receive a detected signal of a heartbeat sensor (21) and a detected signal of an inertial sensor (22); and

a processor (31) configured to estimate a heart rate in case where an exercise intensity obtain from the detected signal of the inertial sensor is equal to or more than a threshold value, based on a relation between the exercise intensity and a heart rate obtained from the detected signal of the heartbeat sensor (21) during a period in which the exercise intensity obtained from the detected signal of the inertial sensor (22) is less than the threshold value.

**2.** A sensor information processing apparatus comprising:

a receiver (34) configured to receive a detected signal of a heartbeat sensor (21) and a detected signal of an inertial sensor (22); and

a processor (31) configured to estimate a heart rate in case where the detected signal of the inertial sensor (22) is equal to or more than a threshold value, based on a relation between the detected signal of the inertial sensor (22) and the detected signal of the heartbeat sensor (21) obtained during a period in which the detected signal of the inertial sensor (22) is less than the threshold value.

**3.** The sensor information processing apparatus according to claim 1 or 2, wherein the processor (31) controls a target frequency band to be processed in the detected signal of the heartbeat sensor (21), according to the estimated heart rate.

**4.** The sensor information processing apparatus according to claim 3, wherein the processor (31) sets a center frequency of the frequency band to a frequency corresponding to the estimated heart rate.

**5.** The sensor information processing apparatus according to claim 4, wherein the processor (31) sets a bandwidth of the frequency band to be narrowed as the estimated heart rate increases.

**6.** The sensor information processing apparatus according to claim 1, wherein the processor (31) calculates a walking cadence based on the detected signal of the inertial sensor (22), and calculates the exercise intensity based on the walking cadence.

**7.** The sensor information processing apparatus according to claim 3, wherein the processor (31) controls the frequency band based on the detected signal of the heartbeat sensor (21), when the estimated heart rate is less than a predetermined heart rate.

**8.** The sensor information processing apparatus according to any one of claims 3 to 6, wherein the processor (31) delays a timing to control the frequency band with respect to an estimated timing of the heart rate.

**9.** The sensor information processing apparatus according to any one of claims 1 to 8, wherein the inertial sensor (22) is a wireless sensor.

**10.** The sensor information processing apparatus according to any one of claims 1 to 9, wherein the heartbeat sensor (21) and the inertial sensor (22) are provided in a sensor unit (2).

**11.** A sensor unit comprising:

a heartbeat sensor (21);
an inertial sensor (22); and
a processor (31) configured to estimate a heart rate in case where an exercise intensity obtain from the detected signal of the inertial sensor is equal to or more than a threshold value, based on a relation between the exercise intensity and a heart rate obtained from the detected signal of the heartbeat sensor (21) during a period in which the exercise intensity obtained from the detected signal of the inertial sensor (22) is less than the threshold value.

**12.** A sensor information processing program for causing a computer to execute a process comprising:

receiving a detected signal of a heartbeat sensor (21) and a detected signal of an inertial sensor (22); and
estimating a heart rate in case where an exercise intensity obtain from the detected signal of the inertial sensor is equal to or more than a threshold value, based on a relation between the exercise intensity and a heart rate obtained from the detected signal of the heartbeat sensor (21) during a period in which the exercise intensity obtained from the detected signal of the inertial sensor (22) is less than the threshold value.

FIG. 1

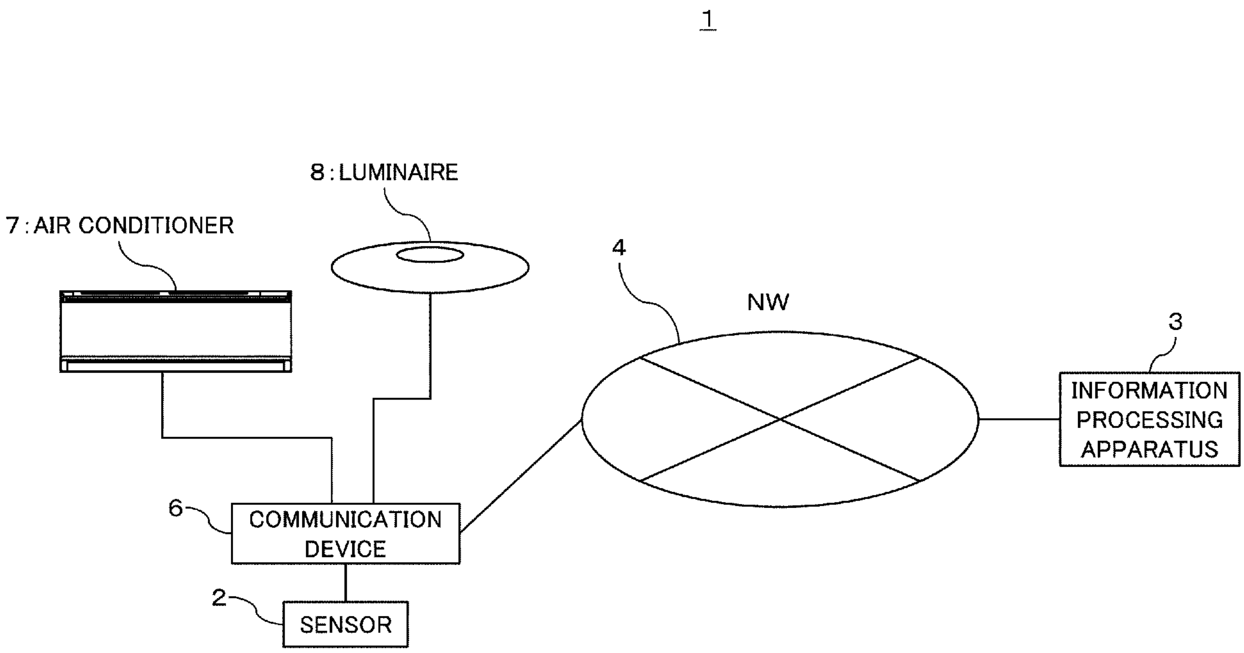
1
8 : LUMINAIRE
7 : AIR CONDITIONER
4
NW
3
INFORMATION PROCESSING APPARATUS
6
COMMUNICATION DEVICE
2
SENSOR

FIG. 2

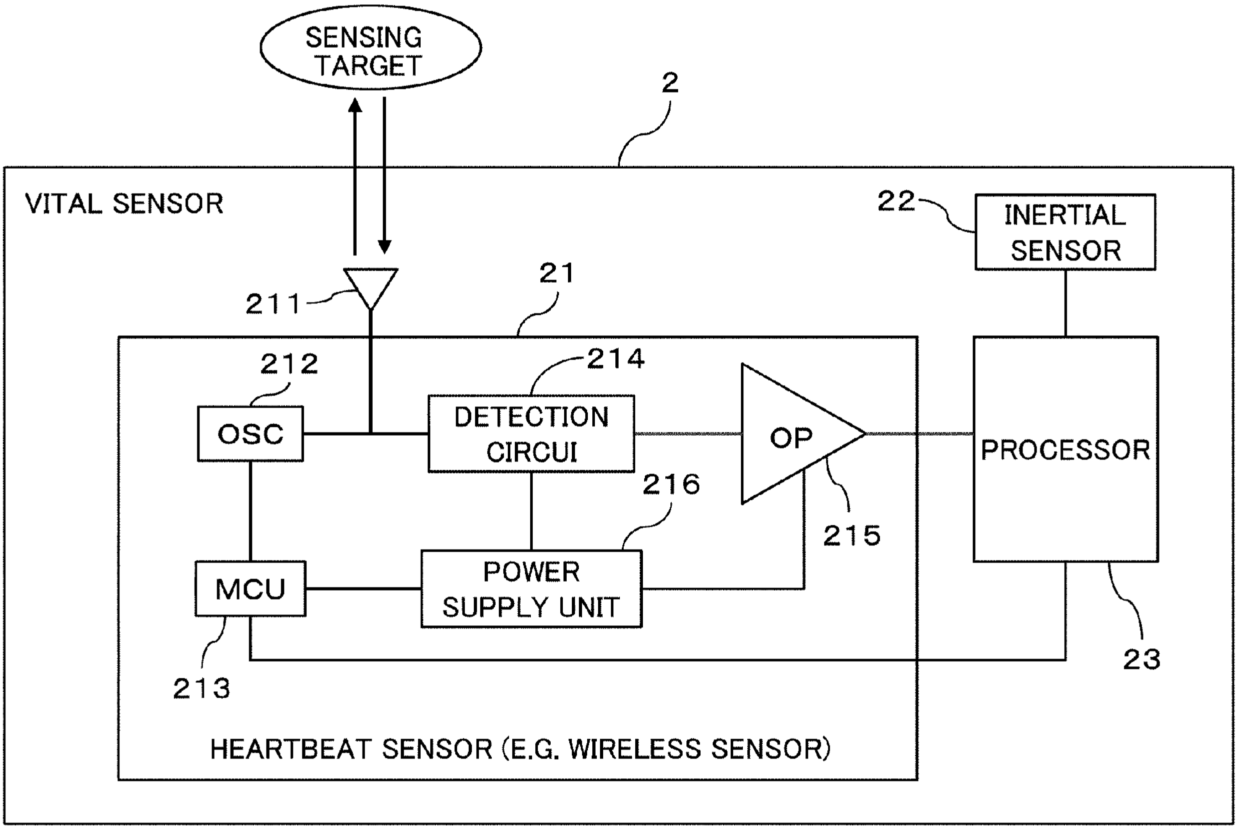
SENSING TARGET
2
VITAL SENSOR
22
INERTIAL SENSOR
211
21
212
214
OSC
DETECTION CIRCUI
OP
PROCESSOR
216
215
MCU
POWER SUPPLY UNIT
213
23
HEARTBEAT SENSOR (E.G. WIRELESS SENSOR)

FIG. 3

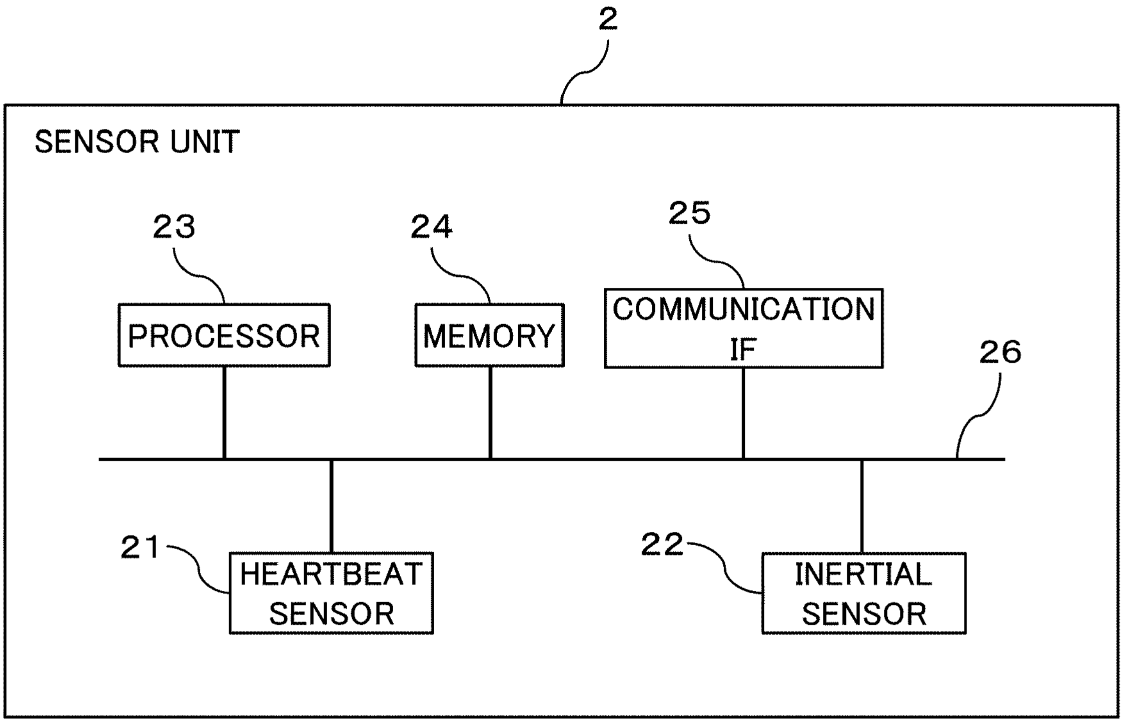
2
SENSOR UNIT
23
PROCESSOR
24
MEMORY
25
COMMUNICATION IF
26
21
HEARTBEAT SENSOR
22
INERTIAL SENSOR

FIG. 4

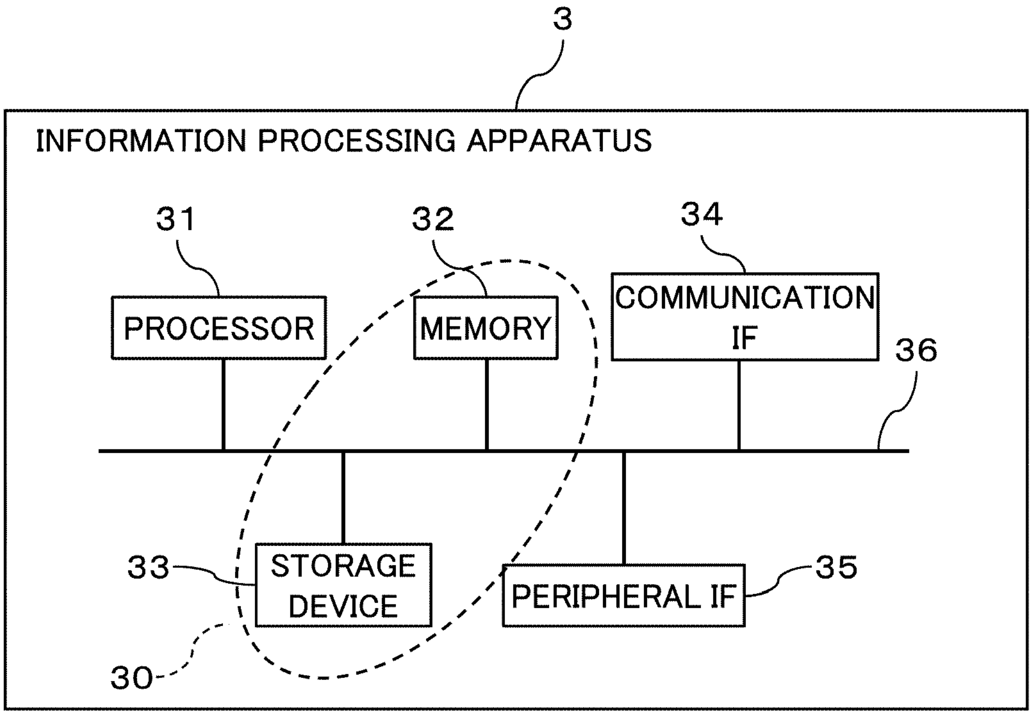
3
INFORMATION PROCESSING APPARATUS
31
PROCESSOR
32
MEMORY
34
COMMUNICATION IF
36
33
STORAGE DEVICE
30
PERIPHERAL IF
35

FIG. 5

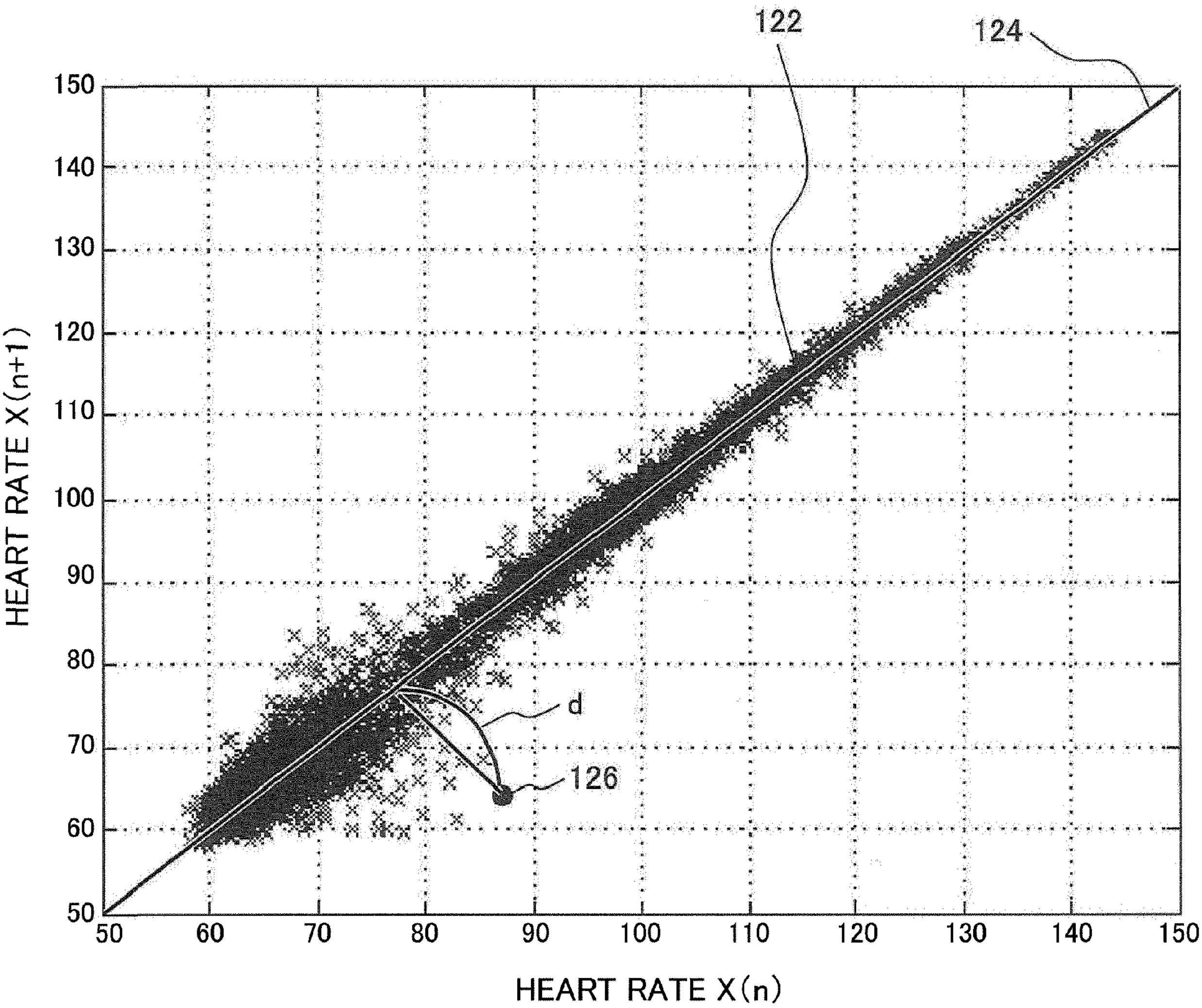
122
124
150
140
130
120
110
100
90
80
70
60
50
50
60
70
80
90
100
110
120
130
140
150
HEART RATE X(n+1)
HEART RATE X(n)
d
126

FIG. 6

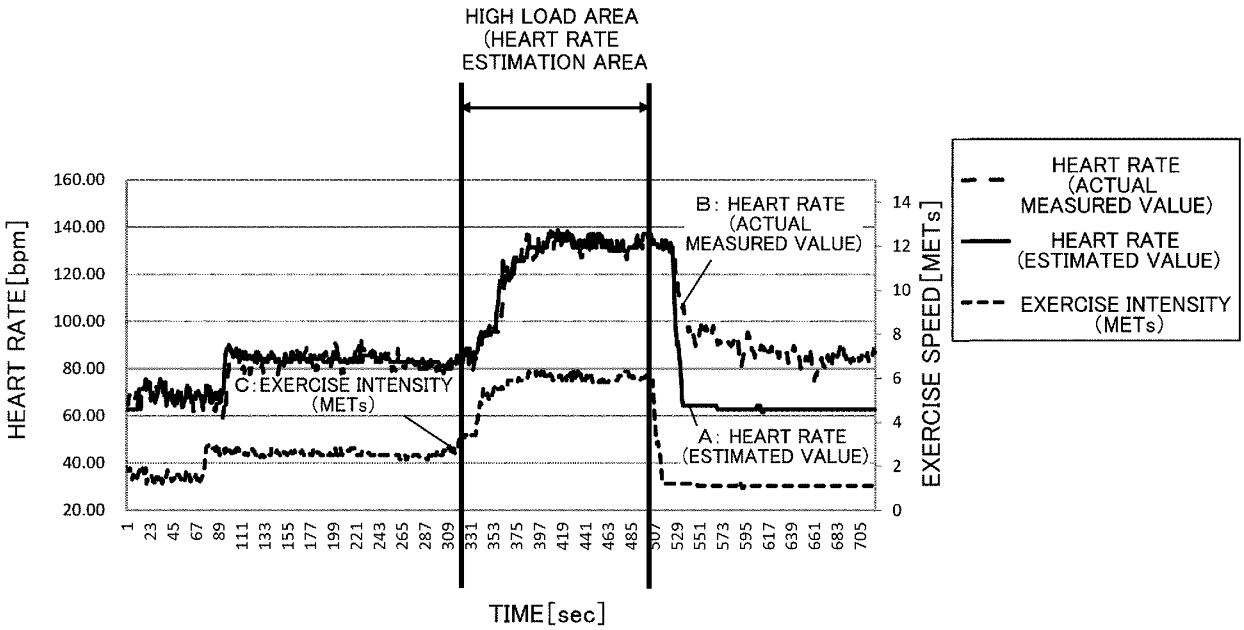
HIGH LOAD AREA
(HEART RATE
ESTIMATION AREA
HEART RATE[bpm]
160.00
140.00
120.00
100.00
80.00
60.00
40.00
20.00
EXERCISE SPEED[METs]
14
12
10
8
6
4
2
0
B: HEART RATE
(ACTUAL
MEASURED VALUE)
C: EXERCISE INTENSITY
(METs)
A: HEART RATE
(ESTIMATED VALUE)
HEART RATE
(ACTUAL
MEASURED VALUE)
HEART RATE
(ESTIMATED VALUE)
EXERCISE INTENSITY
(METs)
1 23 45 67 89 111 133 155 177 199 221 243 265 287 309 331 353 375 397 419 441 463 485 507 529 551 573 595 617 639 661 683 705
TIME[sec]

FIG. 7

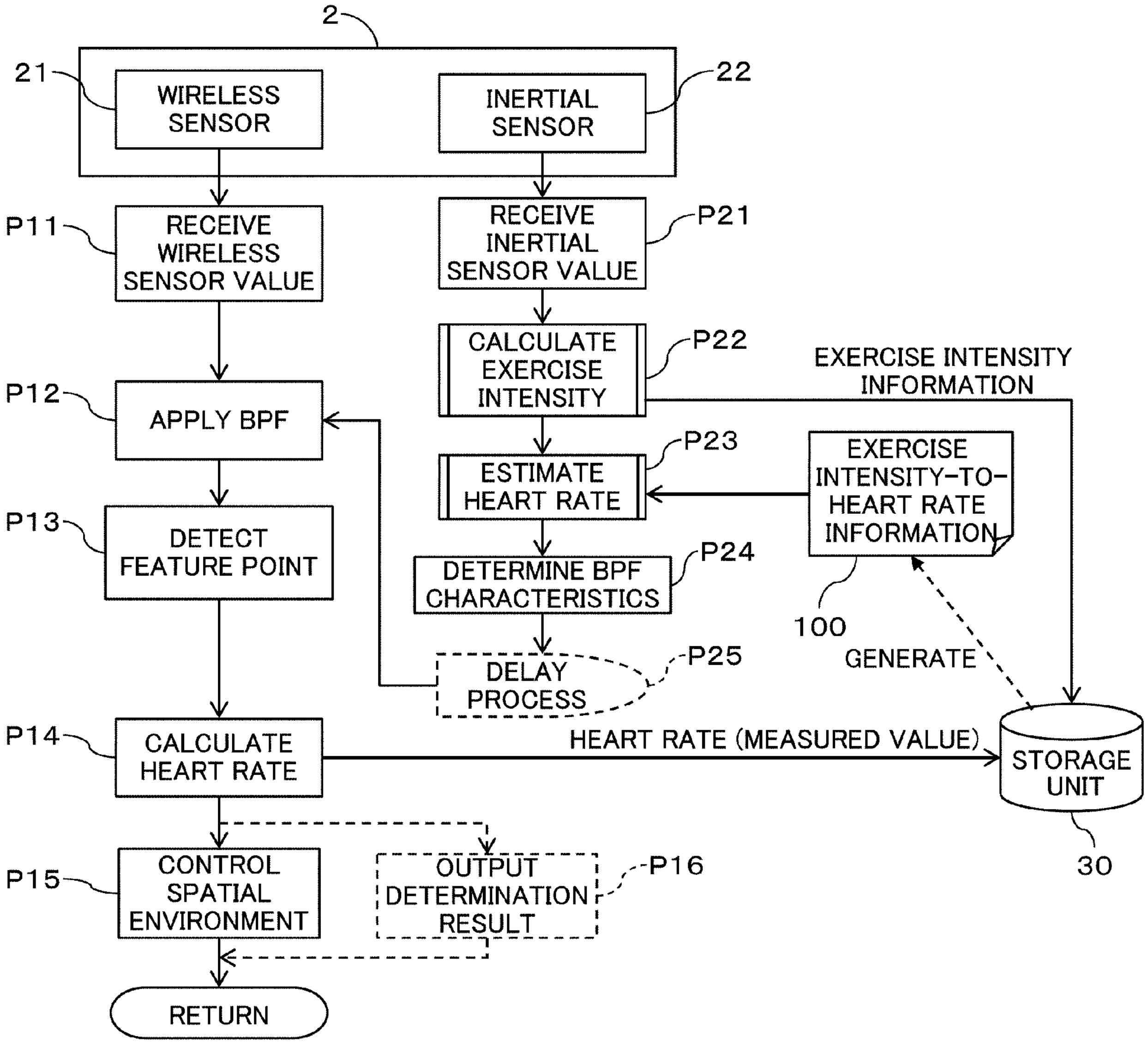
2
21
WIRELESS SENSOR
INERTIAL SENSOR
22
P11
RECEIVE WIRELESS SENSOR VALUE
RECEIVE INERTIAL SENSOR VALUE
P21
CALCULATE EXERCISE INTENSITY
P22
EXERCISE INTENSITY INFORMATION
P12
APPLY BPF
P23
ESTIMATE HEART RATE
EXERCISE INTENSITY-TO-HEART RATE INFORMATION
P13
DETECT FEATURE POINT
DETERMINE BPF CHARACTERISTICS
P24
100
DELAY PROCESS
P25
GENERATE
P14
CALCULATE HEART RATE
HEART RATE (MEASURED VALUE)
STORAGE UNIT
30
P15
CONTROL SPATIAL ENVIRONMENT
OUTPUT DETERMINATION RESULT
P16
RETURN

FIG. 8

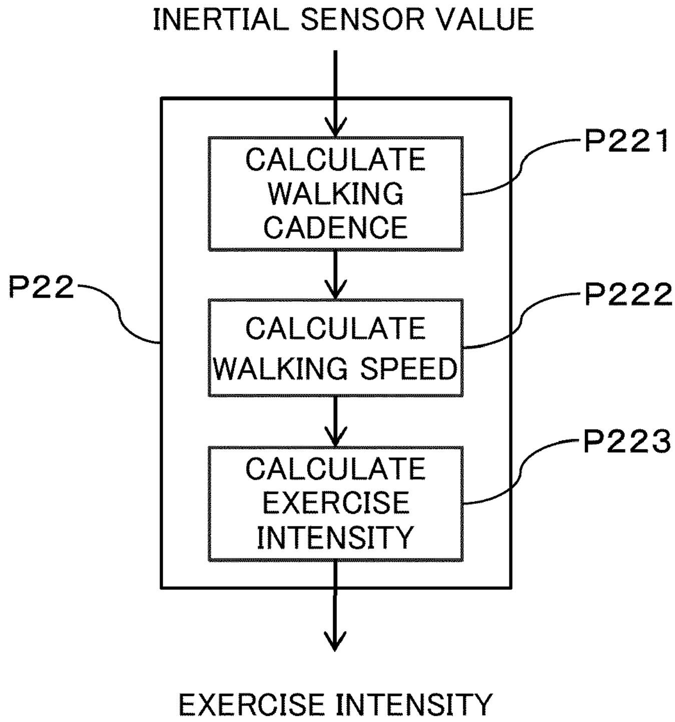
INERTIAL SENSOR VALUE
CALCULATE WALKING CADENCE
P221
P22
CALCULATE WALKING SPEED
P222
CALCULATE EXERCISE INTENSITY
P223
EXERCISE INTENSITY

FIG. 9

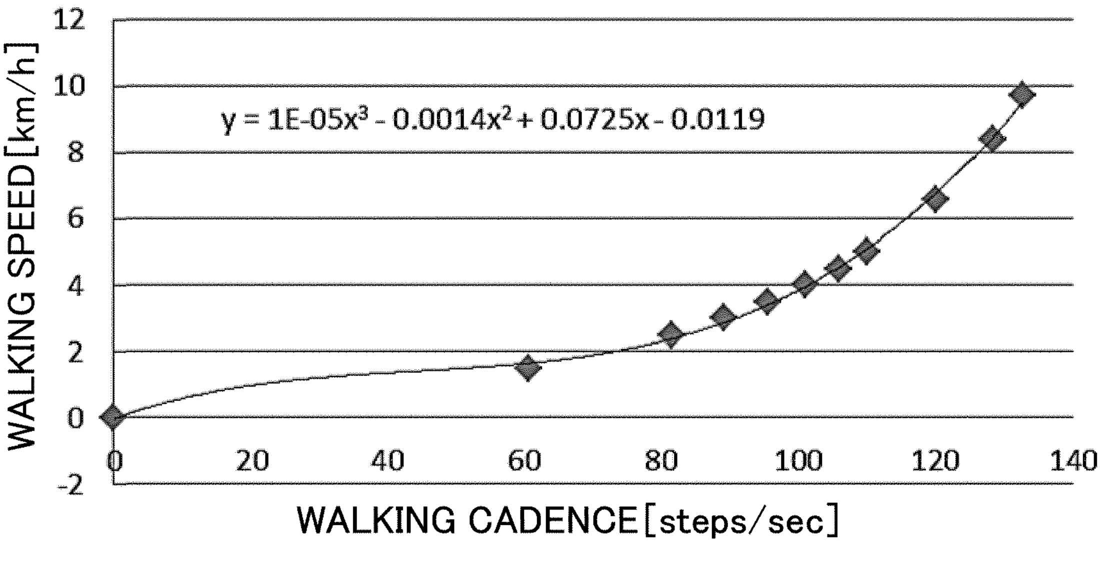
12
10
8
6
4
2
0
-2
0
20
40
60
80
100
120
140
y = 1E-05x³ - 0.0014x² + 0.0725x - 0.0119
WALKING SPEED[km/h]
WALKING CADENCE[steps/sec]

FIG. 10

| METs | km/h |
|---|---|
| 1.0 | 0.0 |
| 1.5 | 1.5 |
| 3.0 | 2.5 |
| 3.3 | 3.0 |
| 3.8 | 3.5 |
| 5.0 | 4.0 |
| 6.3 | 4.5 |
| 7.0 | 5.0 |
| 8.0 | 6.6 |
| 9.0 | 8.4 |
| 10.0 | 9.7 |
| 11.0 | 10.8 |
| 15.0 | 14.5 |

FIG. 11

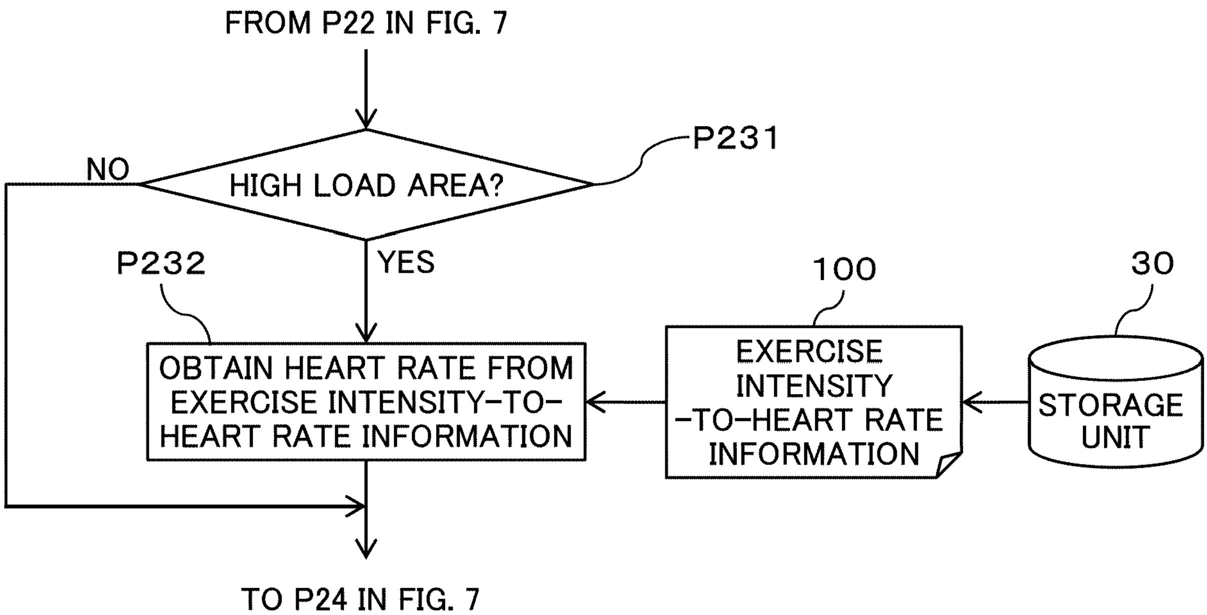
FROM P22 IN FIG. 7
P231
NO
HIGH LOAD AREA?
YES
P232
100
30
OBTAIN HEART RATE FROM EXERCISE INTENSITY-TO-HEART RATE INFORMATION
EXERCISE INTENSITY -TO-HEART RATE INFORMATION
STORAGE UNIT
TO P24 IN FIG. 7

FIG. 12

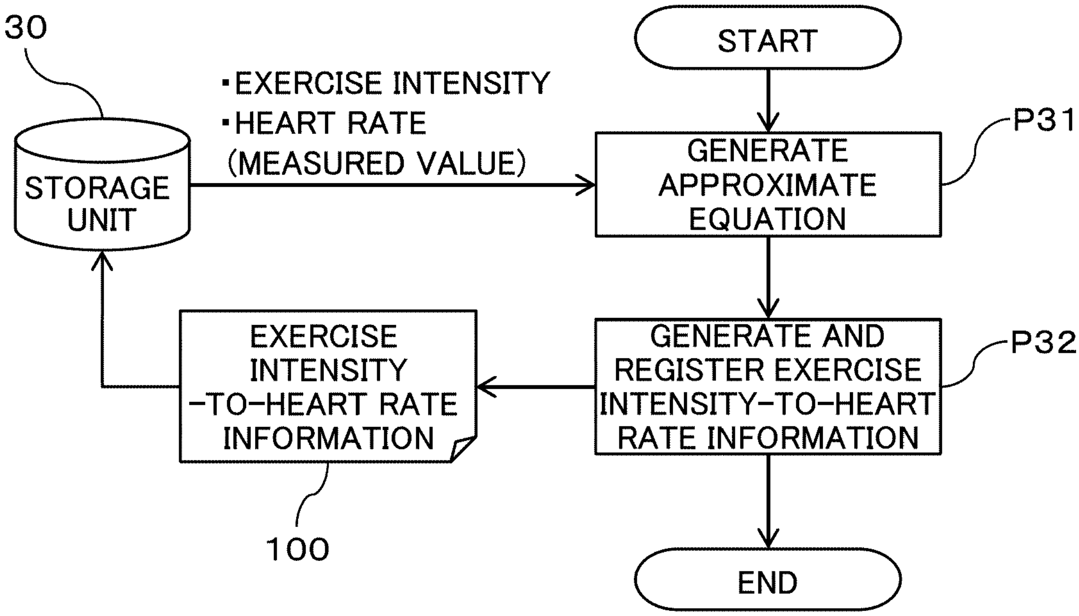
30
STORAGE UNIT
・EXERCISE INTENSITY
・HEART RATE
(MEASURED VALUE)
START
GENERATE APPROXIMATE EQUATION
P31
GENERATE AND REGISTER EXERCISE INTENSITY-TO-HEART RATE INFORMATION
P32
EXERCISE INTENSITY -TO-HEART RATE INFORMATION
100
END

FIG. 13

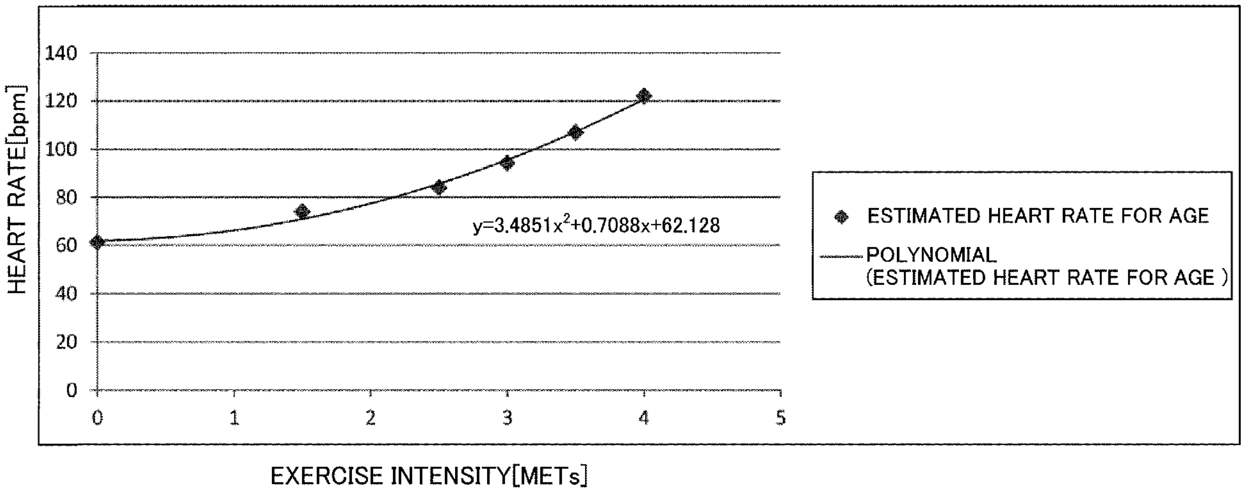
HEART RATE[bpm]
140
120
100
80
60
40
20
0
0
1
2
3
4
5
EXERCISE INTENSITY[METs]
y=3.4851x²+0.7088x+62.128
ESTIMATED HEART RATE FOR AGE
POLYNOMIAL
(ESTIMATED HEART RATE FOR AGE )

FIG. 14

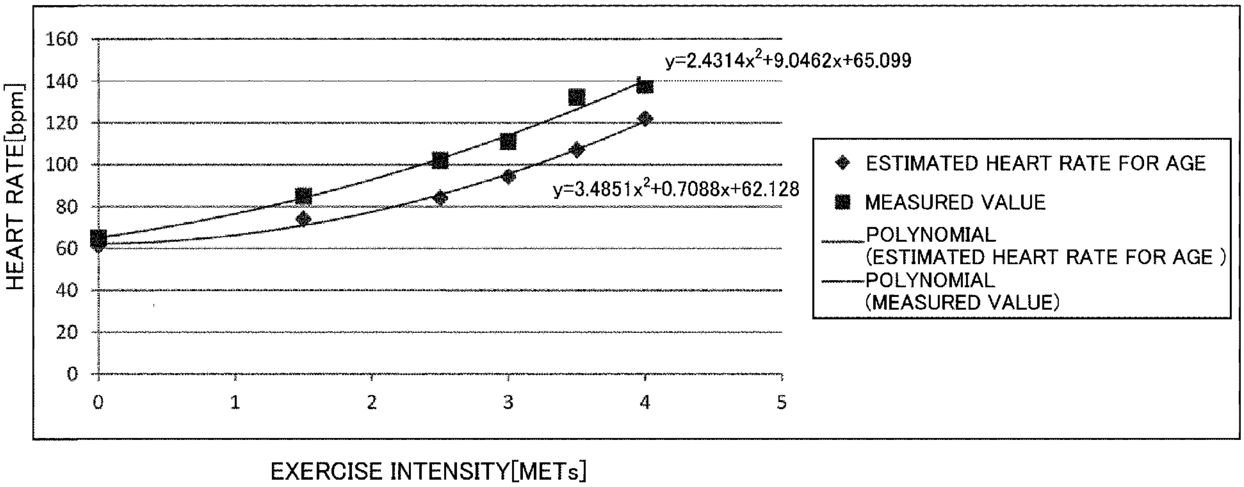
160
140
120
100
80
60
40
20
0
HEART RATE[bpm]
0
1
2
3
4
5
y=2.4314x^2+9.0462x+65.099
y=3.4851x^2+0.7088x+62.128
ESTIMATED HEART RATE FOR AGE
MEASURED VALUE
POLYNOMIAL
(ESTIMATED HEART RATE FOR AGE )
POLYNOMIAL
(MEASURED VALUE)
EXERCISE INTENSITY[METs]

FIG. 15

| METs | WALKING SPEED(km/h) | ESTIMATED HEART RATE FOR AGE [bpm] | MEASURED HEART RATE[bpm] |
|---|---|---|---|
| 1.0 | 0.0 | 63.2 | 65 |
| 1.5 | 1.5 | 70.3 | 85 |
| 3.0 | 2.5 | 91.6 | 102 |
| 3.3 | 3.0 | 95.86 | 111 |
| 3.8 | 3.5 | 102.96 | 132 |

FIG. 16A

| METs | WALKING SPEED(km/h) | ESTIMATED HEART RATE FOR AGE [bpm] | MEASURED HEART RATE[bpm] | CONSTANT S |
|---|---|---|---|---|
| 1.0 | 0.0 | 63.2 | 65 | 227 |
| 1.5 | 1.5 | 70.3 | 85 | 300.3333 |
| 3.0 | 2.5 | 91.6 | 102 | 230.3333 |
| 3.3 | 3.0 | 95.86 | 111 | 240.9394 |
| 3.8 | 3.5 | 102.96 | 132 | 270.6842 |

FIG. 16B

| METs | WALKING SPEED(km/h) | ESTIMATED HEART RATE FOR AGE [bpm] | MEASURED HEART RATE[bpm] | CONSTANT S | ESTIMATED HEART RATE[bpm] |
|---|---|---|---|---|---|
| 1.0 | 0.0 | 63.2 | 65 | 227 | 65 |
| 1.5 | 1.5 | 70.3 | 85 | 300.3333 | 85 |
| 3.0 | 2.5 | 91.6 | 102 | 230.3333 | 102 |
| 3.3 | 3.0 | 95.86 | 111 | 240.9394 | 111 |
| 3.8 | 3.5 | 102.96 | 132 | 270.6842 | 132 |
| 5.0 | 4.0 | 120 | – | 240.384 | 143.692 |
| 6.3 | 4.5 | 138.46 | – | 223.2616 | 158.0448219 |
| 7.0 | 5.0 | 148.4 | – | 211.4908 | 162.14356 |
| 8.0 | 6.6 | 162.6 | 162.6 | 191.5 | 162.6 |

FIG. 17

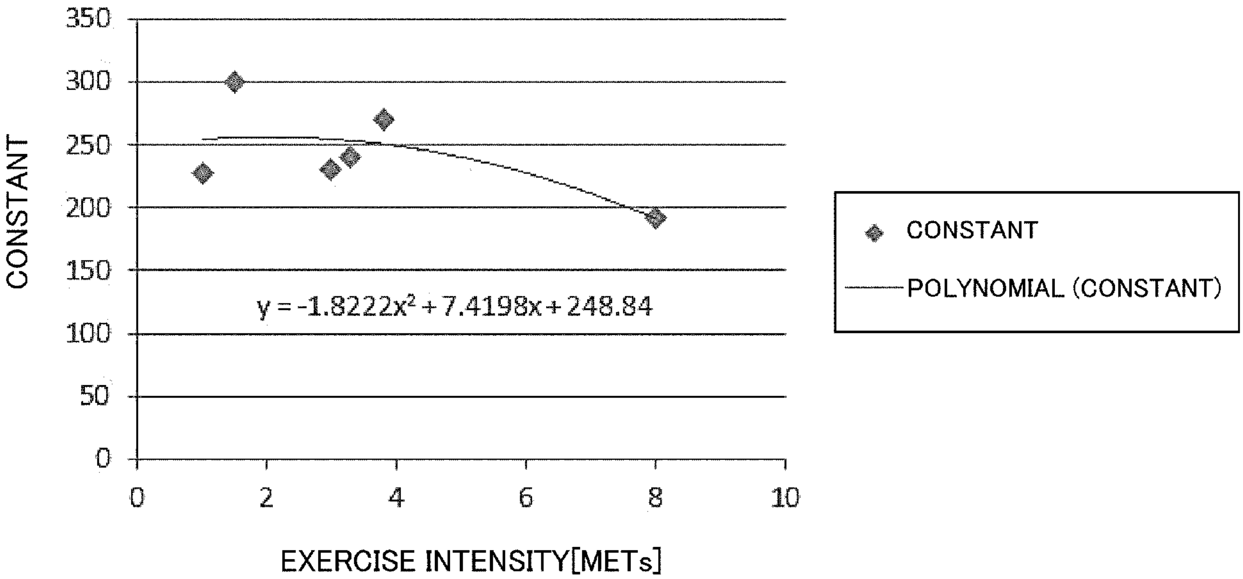
350
300
250
200
150
100
50
0
CONSTANT
y = -1.8222x² + 7.4198x + 248.84
0
2
4
6
8
10
EXERCISE INTENSITY[METs]
CONSTANT
POLYNOMIAL (CONSTANT)

FIG. 18

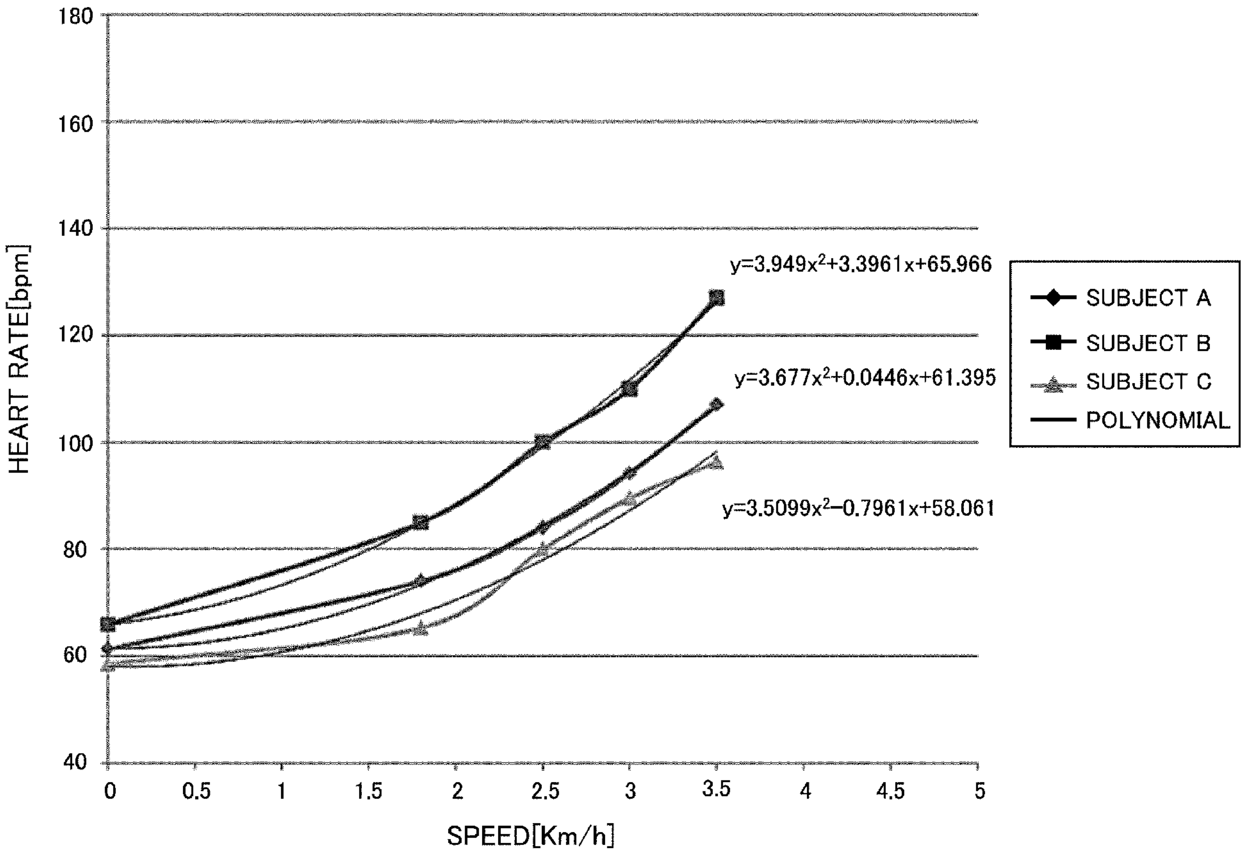
180
160
140
120
100
80
60
40
HEART RATE[bpm]
0
0.5
1
1.5
2
2.5
3
3.5
4
4.5
5
SPEED[Km/h]
y=3.949x2+3.3961x+65.966
y=3.677x2+0.0446x+61.395
y=3.5099x2−0.7961x+58.061
SUBJECT A
SUBJECT B
SUBJECT C
POLYNOMIAL

FIG. 19

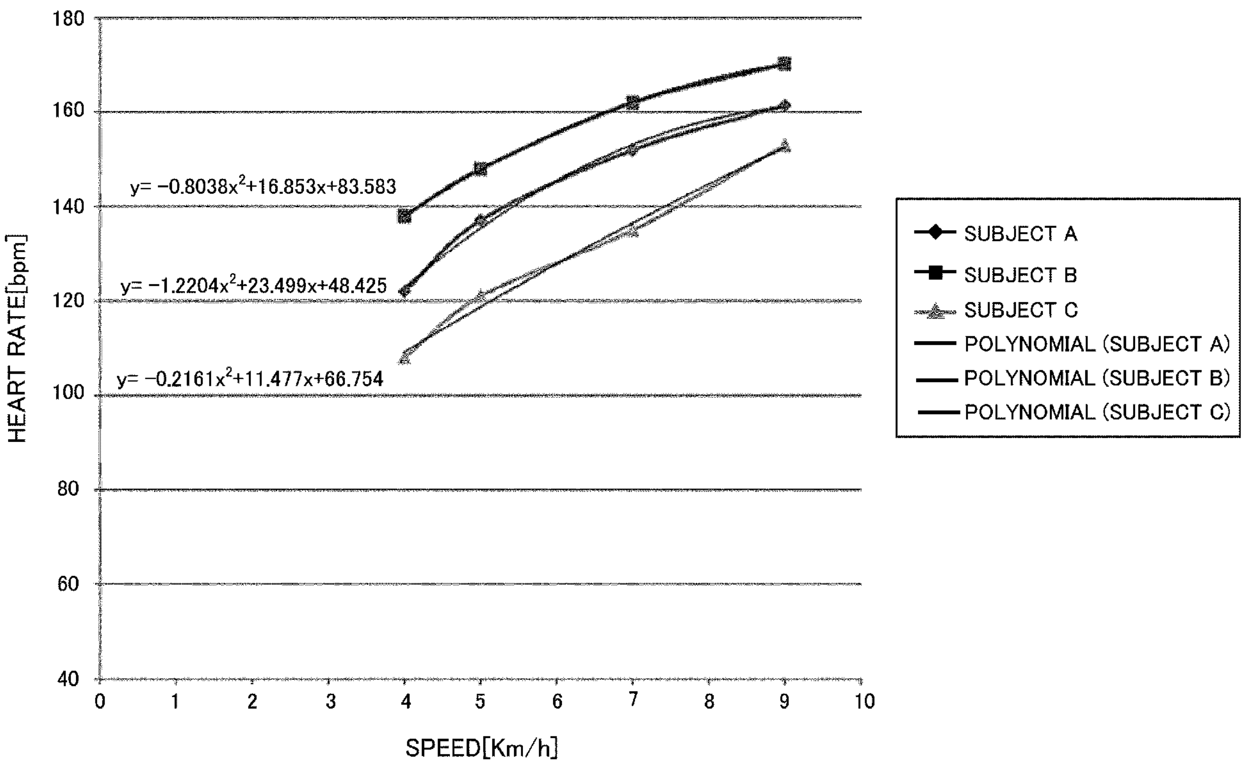
180
160
140
120
100
80
60
40
0
1
2
3
4
5
6
7
8
9
10
HEART RATE[bpm]
SPEED[Km/h]
y= −0.8038x²+16.853x+83.583
y= −1.2204x²+23.499x+48.425
y= −0.2161x²+11.477x+66.754
SUBJECT A
SUBJECT B
SUBJECT C
POLYNOMIAL (SUBJECT A)
POLYNOMIAL (SUBJECT B)
POLYNOMIAL (SUBJECT C)

FIG. 20

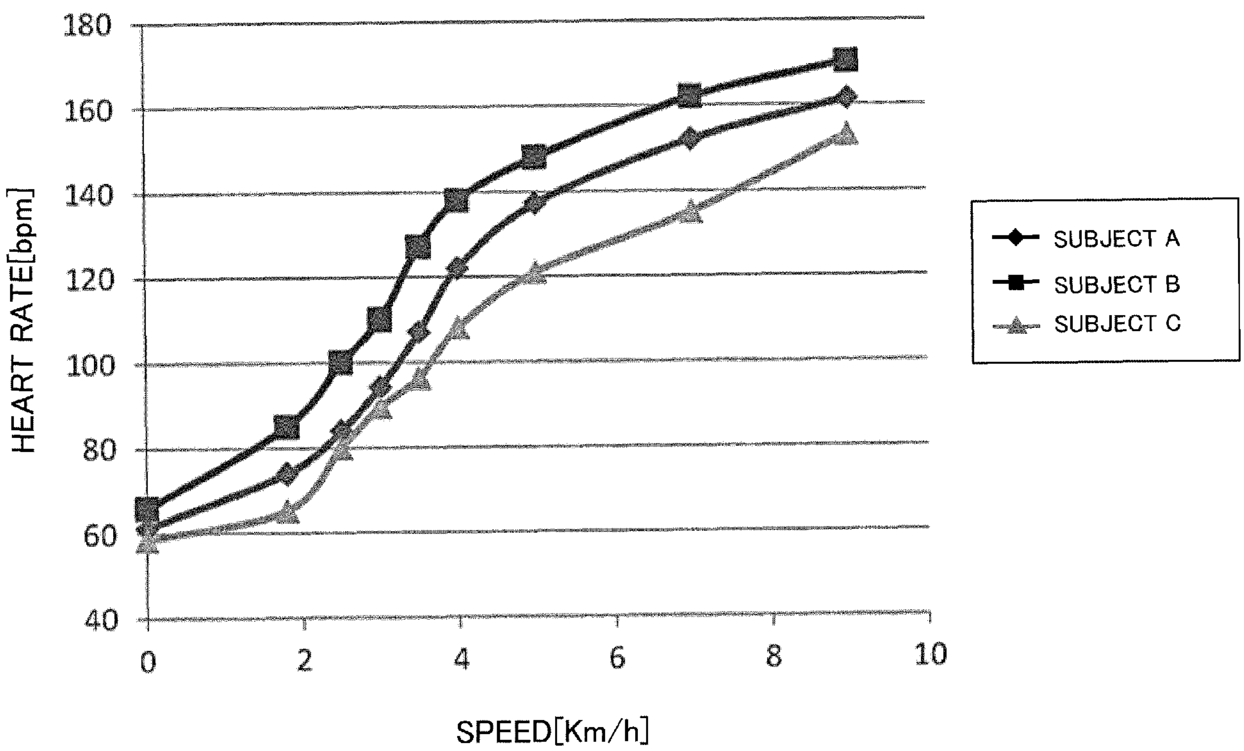
180
160
140
120
100
80
60
40
HEART RATE[bpm]
0
2
4
6
8
10
SPEED[Km/h]
SUBJECT A
SUBJECT B
SUBJECT C

FIG. 21

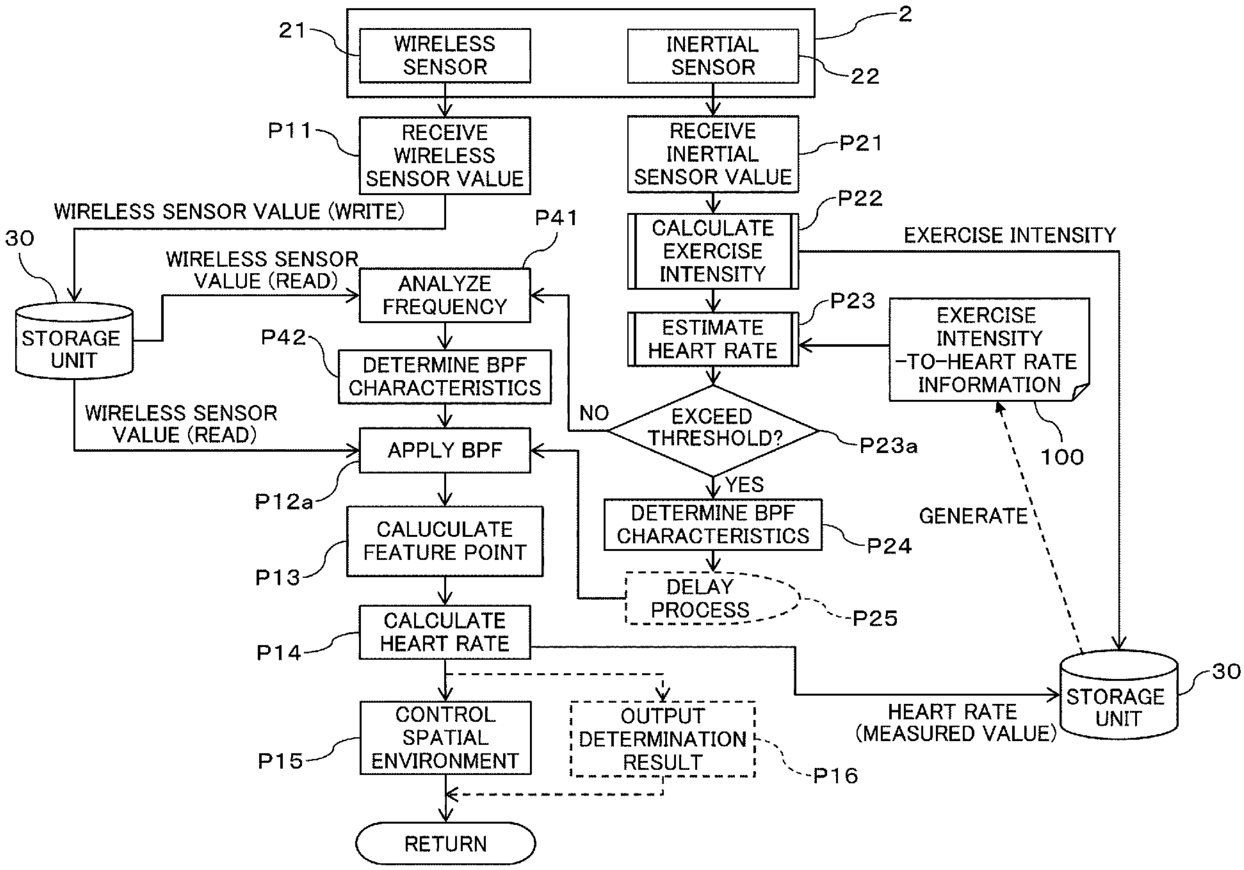
2
21
WIRELESS SENSOR
INERTIAL SENSOR
22
P11
RECEIVE WIRELESS SENSOR VALUE
RECEIVE INERTIAL SENSOR VALUE
P21
WIRELESS SENSOR VALUE (WRITE)
P22
30
P41
CALCULATE EXERCISE INTENSITY
EXERCISE INTENSITY
WIRELESS SENSOR VALUE (READ)
ANALYZE FREQUENCY
P23
STORAGE UNIT
P42
ESTIMATE HEART RATE
EXERCISE INTENSITY -TO-HEART RATE INFORMATION
DETERMINE BPF CHARACTERISTICS
WIRELESS SENSOR VALUE (READ)
NO
EXCEED THRESHOLD?
P23a
100
APPLY BPF
P12a
YES
DETERMINE BPF CHARACTERISTICS
GENERATE
CALUCULATE FEATURE POINT
P24
P13
DELAY PROCESS
P25
CALCULATE HEART RATE
P14
30
HEART RATE (MEASURED VALUE)
STORAGE UNIT
CONTROL SPATIAL ENVIRONMENT
OUTPUT DETERMINATION RESULT
P15
P16
RETURN

FIG. 22

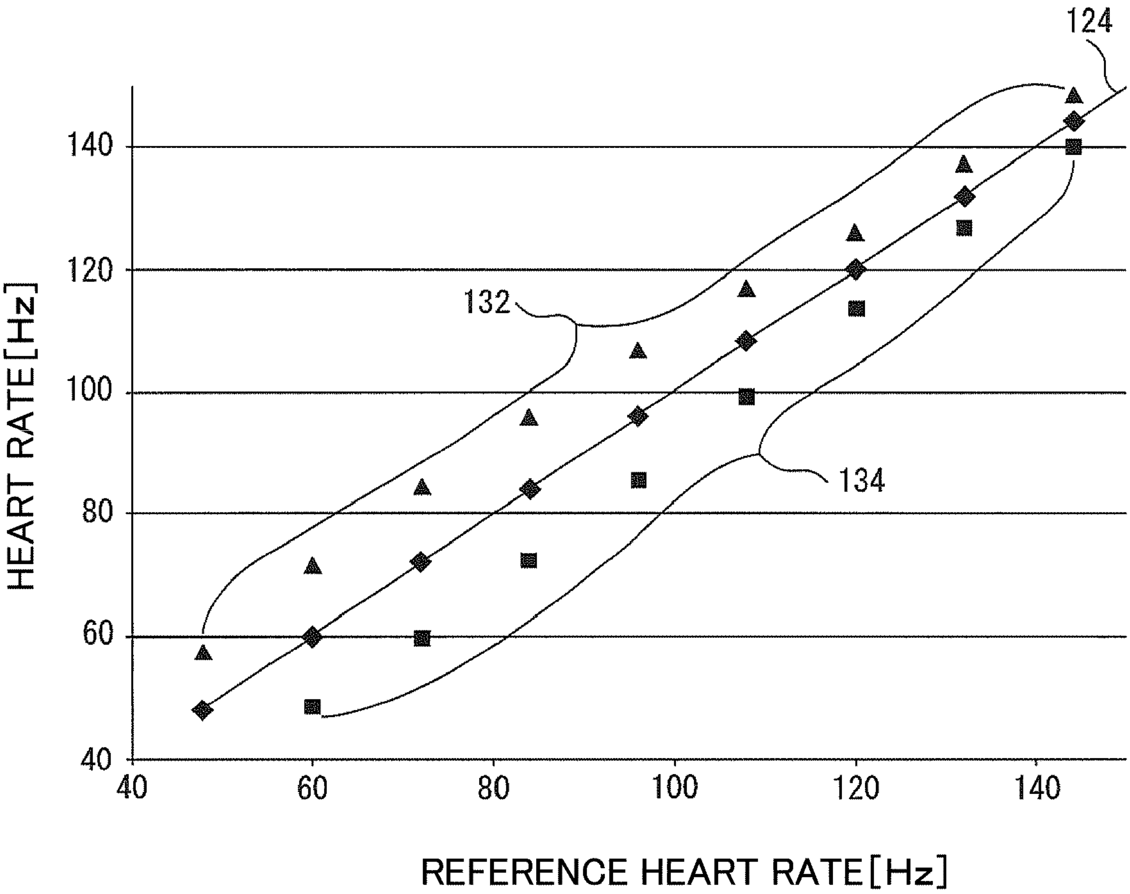
124
132
134
HEART RATE[Hz]
140
120
100
80
60
40
40
60
80
100
120
140
REFERENCE HEART RATE[Hz]

FIG. 23

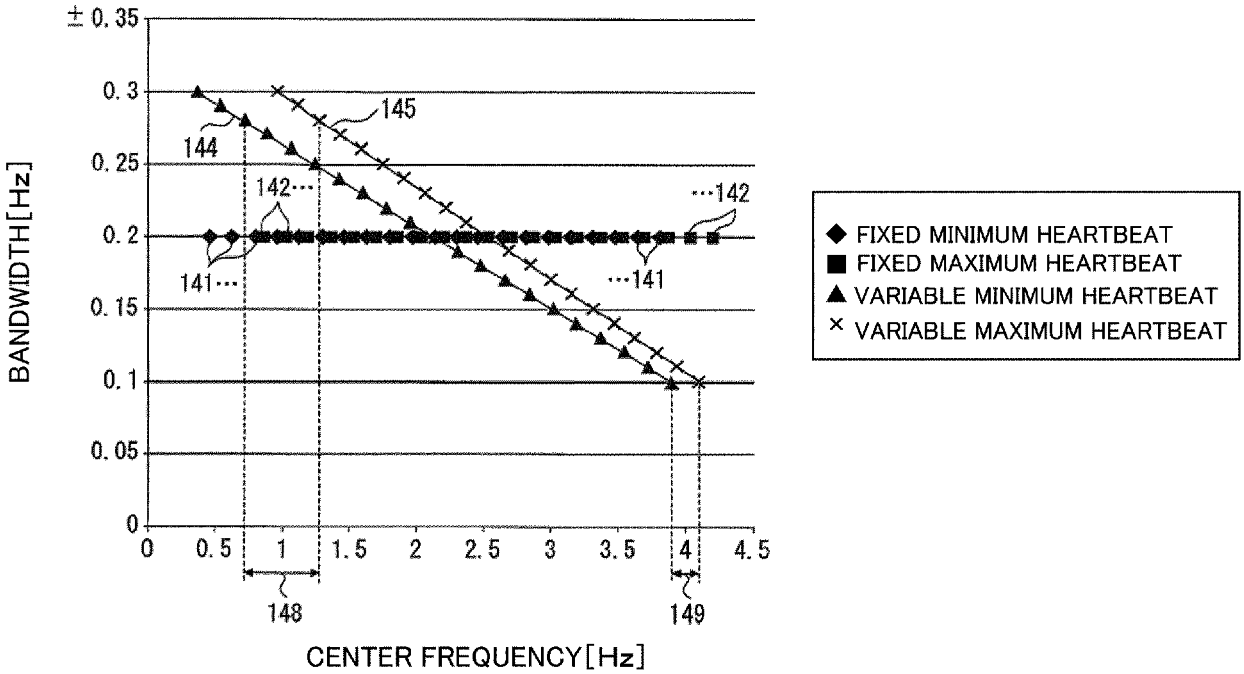
±0.35
0.3
0.25
0.2
0.15
0.1
0.05
0
BANDWIDTH[Hz]
0
0.5
1
1.5
2
2.5
3
3.5
4
4.5
CENTER FREQUENCY[Hz]
144
145
142…
…142
141…
…141
148
149
FIXED MINIMUM HEARTBEAT
FIXED MAXIMUM HEARTBEAT
VARIABLE MINIMUM HEARTBEAT
VARIABLE MAXIMUM HEARTBEAT

FIG. 24

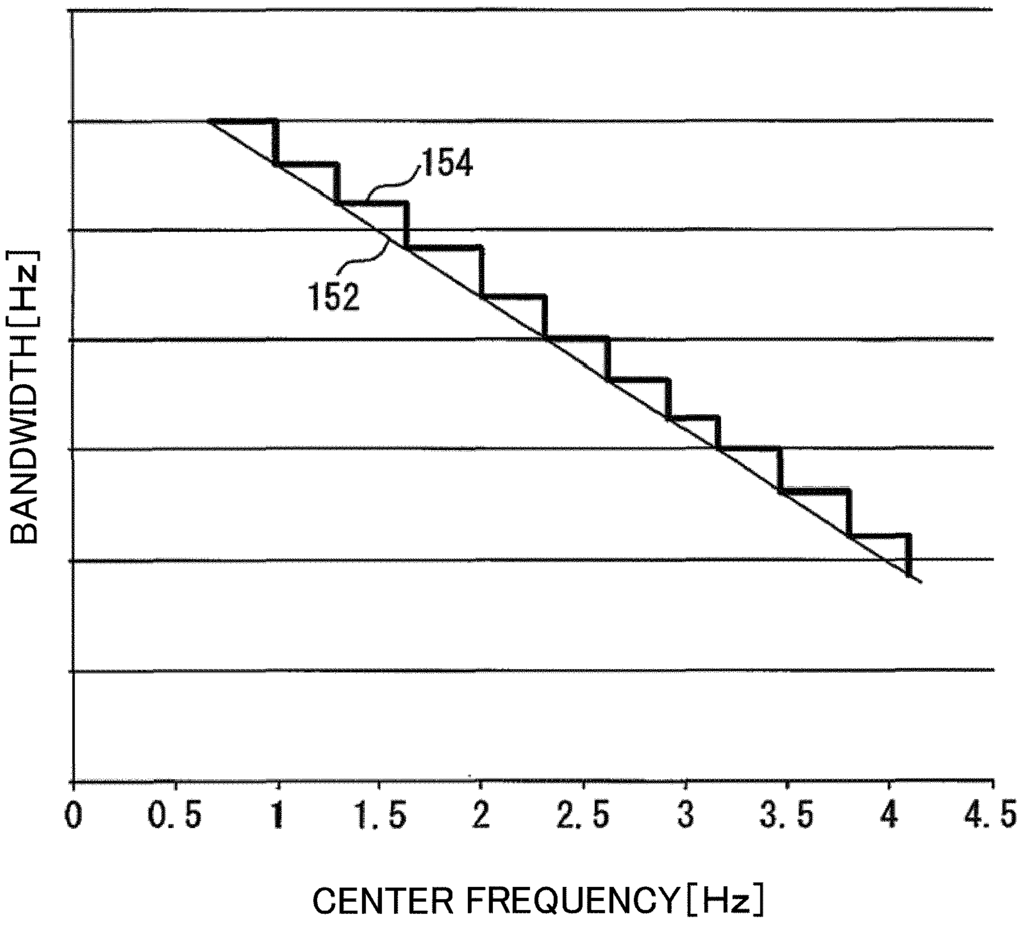

FIG. 25

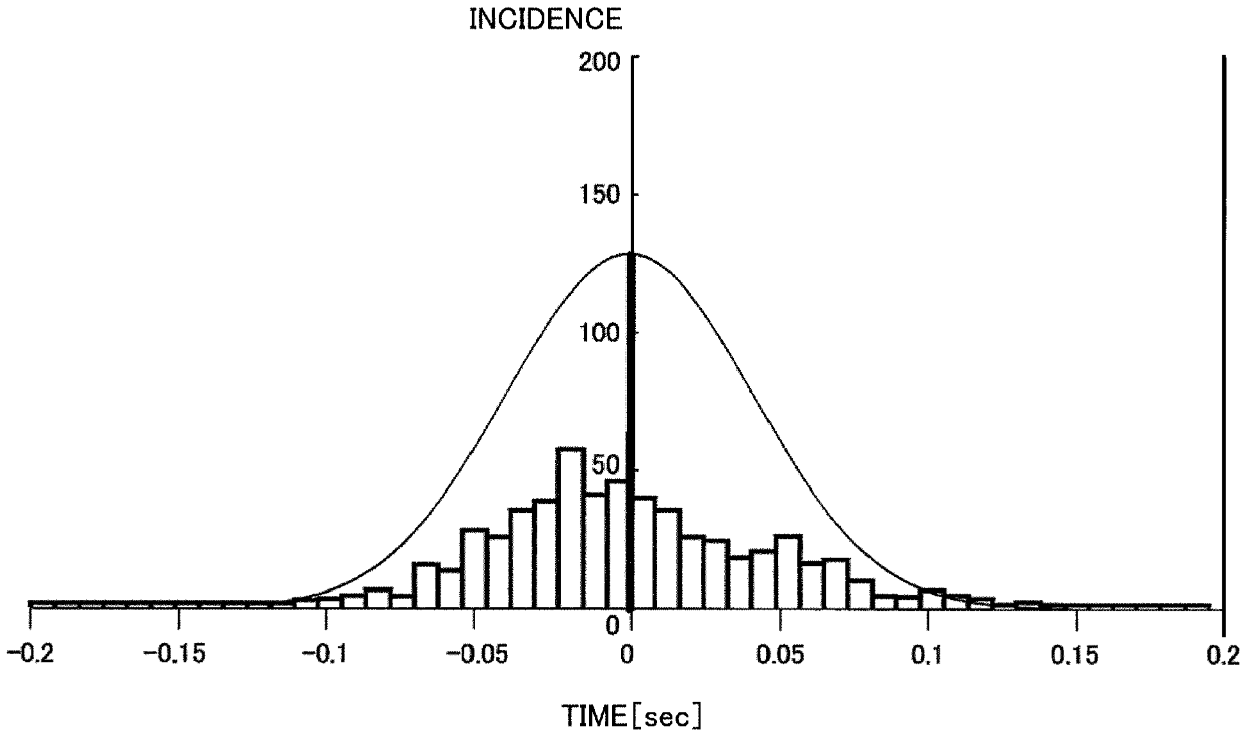

FIG. 26

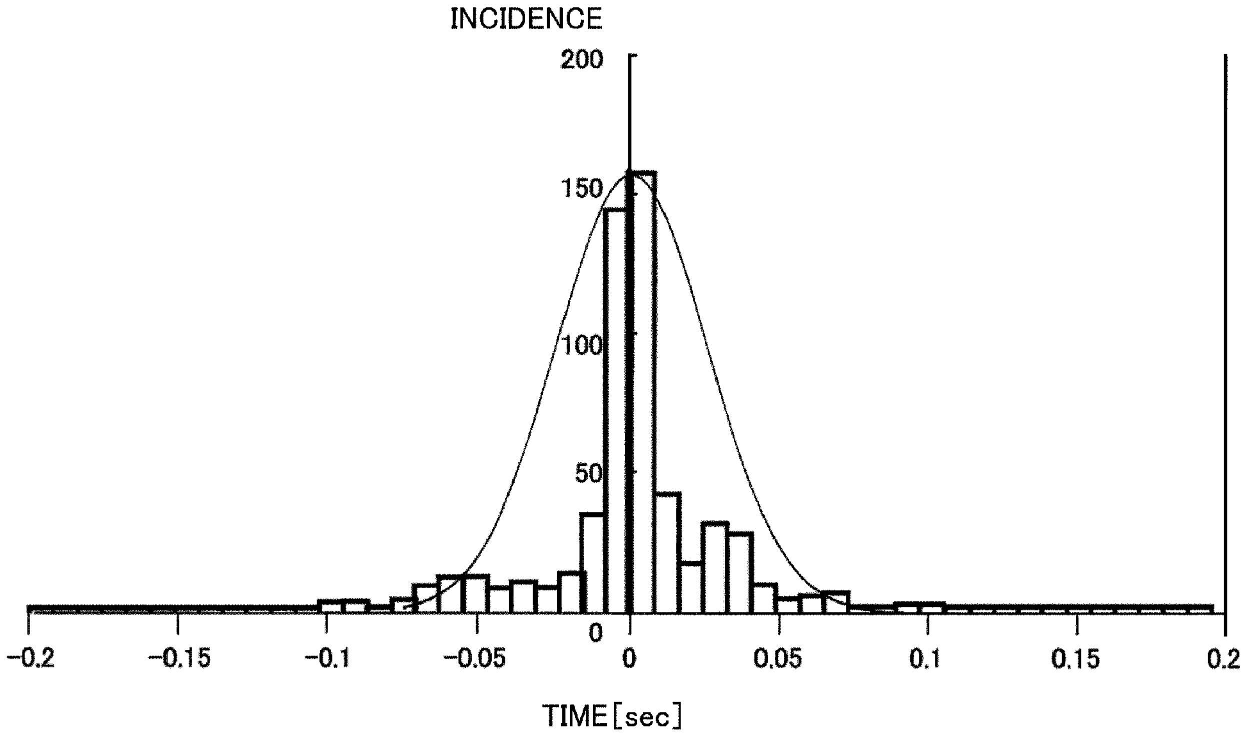

FIG. 27

| | HEART RATE NEAR 60 | HEART RATE NEAR 120 |
|---|---|---|
| THE NUMBER OF SAMPLES | 532 | 532 |
| AVERAGE VALUE Xbar | −0.000652964 | 0.000556391 |
| SAMPLE STANDARD DEVIATION σ | 0.041438026 | 0.025446051 |
| MINIMUM VALUE Min | −0.10593357 | −0.103 |
| MAXIMUM VALUE Max | 0.134123404 | 0.096 |
| RANGE R | 0.240056974 | 0.199 |
| SKEW | 0.383 | −0.571 |
| KURTOSIS | −0.097 | 2.991 |
| BPF LOWER LIMIT | −0.165 | −0.165 |
| BPF UPPER LIMIT | 0.165 | 0.165 |
| CP | 1.33 | 2.16 |
| CPK | 1.32 | 2.15 |

FIG. 28

| | HEART RATE NEAR 60 | HEART RATE NEAR 120 |
|---|---|---|
| THE NUMBER OF SAMPLES | 532 | 532 |
| AVERAGE VALUE Xbar | −0.000652964 | 0.000556391 |
| SAMPLE STANDARD DEVIATION $\sigma$ | 0.041438026 | 0.025446051 |
| MINIMUM VALUE Min | −0.10593357 | −0.103 |
| MAXIMUM VALUE Max | 0.134123404 | 0.096 |
| RANGE R | 0.240056974 | 0.199 |
| SKEW | 0.383 | −0.571 |
| KURTOSIS | −0.097 | 2.991 |
| BPF LOWER LIMIT | −0.1015 | −0.1015 |
| BPF UPPER LIMIT | 0.1015 | 0.1015 |
| CP | 0.82 | 1.33 |
| CPK | 0.81 | 1.32 |

FIG. 29

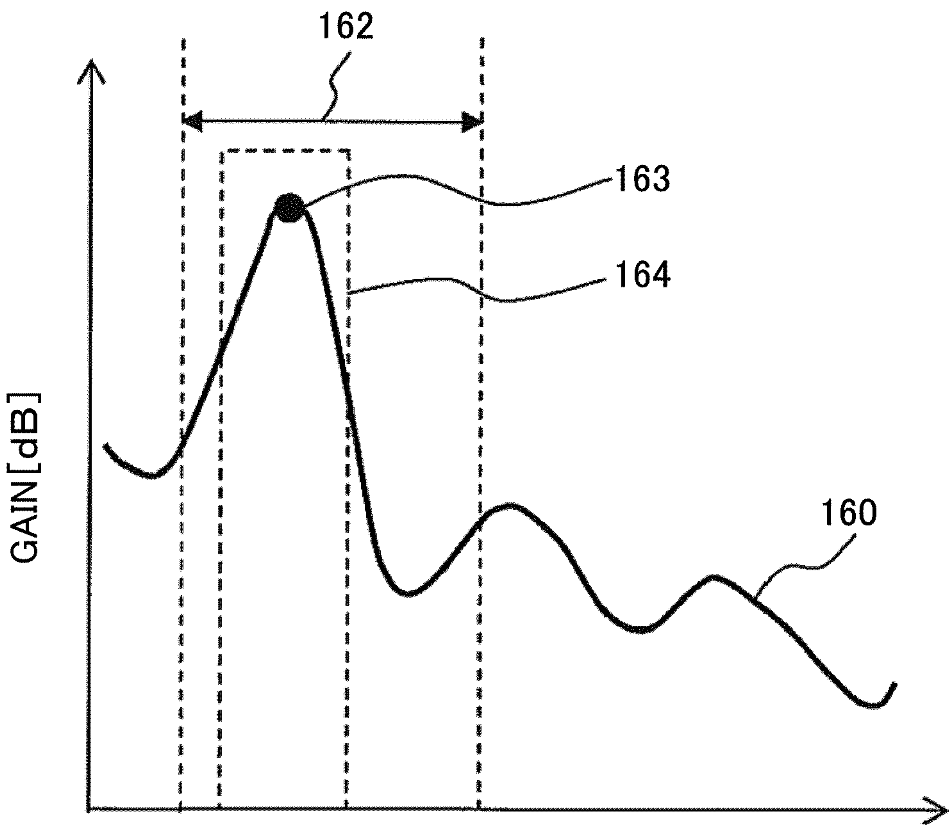

FIG. 30

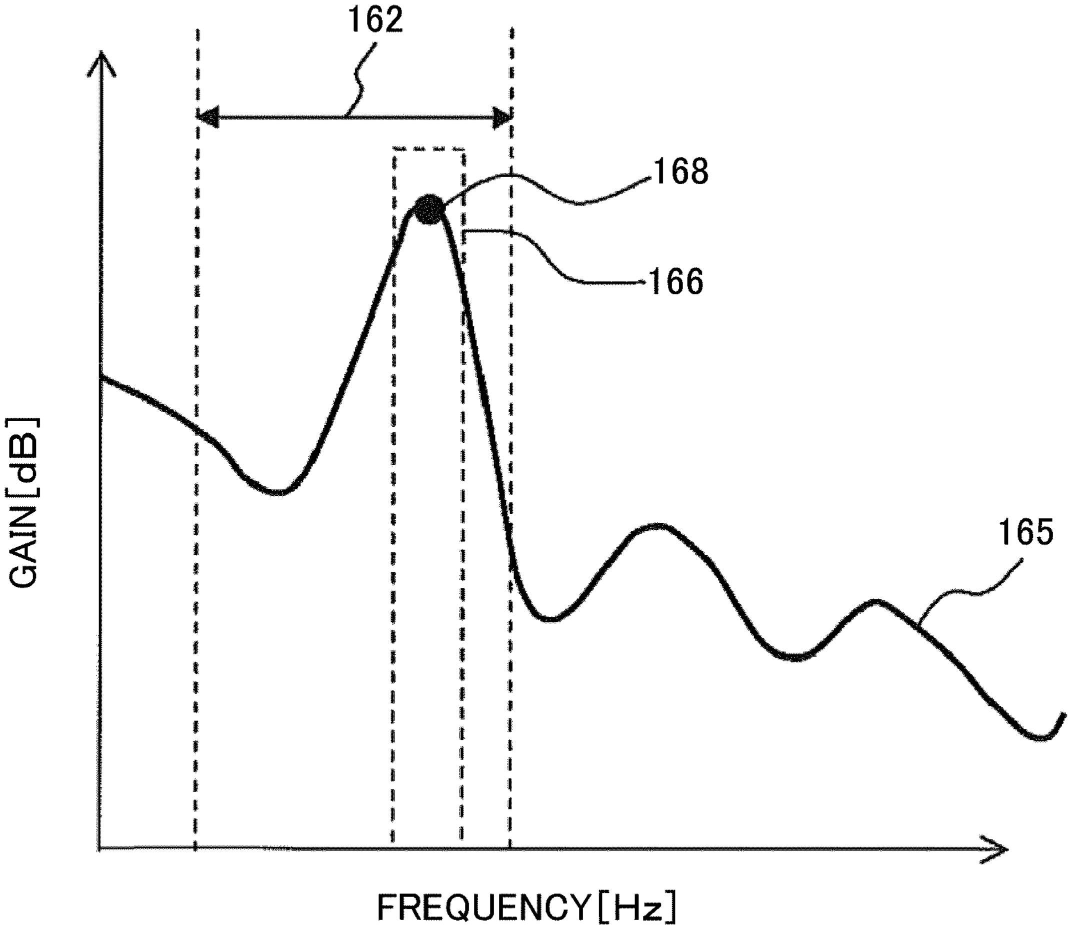

FIG. 31

BANDWIDTH INFORMATION 170

171 172 173

| REFERENCE HEART RATE[bpm] | BPF WIDTH LOWER LIMIT[Hz] | BPF WIDTH UPPER LIMIT[Hz] |
|---|---|---|
| 40 | 0.366666667 | 0.966666667 |
| 50 | 0.543333333 | 1.123333333 |
| 60 | 0.72 | 1.28 |
| 70 | 0.896666667 | 1.436666667 |
| 80 | 1.073333333 | 1.593333333 |
| 90 | 1.25 | 1.75 |
| 100 | 1.426666667 | 1.906666667 |
| 110 | 1.603333333 | 2.063333333 |
| 120 | 1.78 | 2.22 |

Europäisches Patentamt
European Patent Office
Office européen des brevets

# EUROPEAN SEARCH REPORT

Application Number
EP 17 15 3263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/342481 A1 (LIU ZONGYI [US] ET AL) 3 December 2015 (2015-12-03)<br>* abstract; figures 1-6 *<br>* paragraphs [0007] - [0046] *<br>----- | 1-12 | INV.<br>A61B5/02<br>A61B5/0205<br>A61B5/024<br>A61B5/11<br>A61B5/00<br><br>ADD.<br>A61B5/05 |
| X | EP 2 229 880 A1 (SUISSE ELECTRONIQUE MICROTECH [CH]) 22 September 2010 (2010-09-22)<br>* abstract; figures 1,2 *<br>* paragraphs [0002], [0005], [0011] - [0019], [0021] - [0035], [0040], [0043] - [0056] *<br>----- | 1-12 | |
| X<br><br>A | US 2015/011898 A1 (ROMESBURG ERIC DOUGLAS [US]) 8 January 2015 (2015-01-08)<br>* abstract; figures 1-6 *<br>* paragraphs [0016] - [0021], [0036] - [0038] *<br>----- | 1,2,6, 9-12<br>3-5,7,8 | |
| X,D | JP 2014 094043 A (FUJITSU LTD) 22 May 2014 (2014-05-22)<br>* abstract; figures 1-22 *<br>* paragraphs [0002] - [0005], [0007] - [0014], [0016] - [0070] *<br>----- | 1-12 | TECHNICAL FIELDS SEARCHED (IPC)<br>A61B |

The present search report has been drawn up for all claims

2

| Place of search | Date of completion of the search | Examiner |
|---|---|---|
| The Hague | 15 June 2017 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 15 3263

This annex lists the patent family members relating to the patent documents cited in the above-mentioned European search report. The members are as contained in the European Patent Office EDP file on
The European Patent Office is in no way liable for these particulars which are merely given for the purpose of information.

15-06-2017

| Patent document cited in search report | Publication date | Patent family member(s) | Publication date |
|---|---|---|---|
| US 2015342481 A1 | 03-12-2015 | CN 106456022 A | 22-02-2017 |
| | | EP 3148423 A1 | 05-04-2017 |
| | | US 2015342481 A1 | 03-12-2015 |
| | | WO 2015184030 A1 | 03-12-2015 |
| EP 2229880 A1 | 22-09-2010 | NONE | |
| US 2015011898 A1 | 08-01-2015 | CN 104168828 A | 26-11-2014 |
| | | HK 1203800 A1 | 06-11-2015 |
| | | US 2015011898 A1 | 08-01-2015 |
| | | WO 2013109389 A1 | 25-07-2013 |
| JP 2014094043 A | 22-05-2014 | JP 6056389 B2 | 11-01-2017 |
| | | JP 2014094043 A | 22-05-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003334269 A
- JP 11192217 A
- JP 2014094043 A
- JP 2014039666 A